# EUROPEAN PATENT SPECIFICATION

(11) **EP 0 494 887 B1**
(45) Date of publication and mention of the grant of the patent: **25.06.1997**
(21) Application number: 90913989.1
(22) Date of filing: 21.09.1990
(51) Int. Cl.: C12M 3/00

(54) **PROCESS AND APPARATUS FOR THE PRODUCTION OF PHOTOSYNTHETIC MICROBES**
PROZESS UND APPARATUR ZUR PRODUKTION VON PHOTOSYNTHETISIERENDEN MIKROBEN
PROCEDE ET APPAREIL DE PRODUCTION DE MICROBES PHOTOSYNTHETIQUES

(30) Priority: 10.10.1989 US 419522
(43) Date of publication of application: 22.07.1992
(73) Proprietor: AQUASEARCH, INC., Kailua, Kona, Hawaii (US)
(72) Inventor: HUNTLEY, Mark, E., La Jolla, CA 92037 (US); WAHLBERG, Dwight, D., deceased (US); REDALJE, Donald, G., Pass Christian, MS 39571 (US)
(74) Representative: Wilson, Nicholas Martin
(86) International application number: US9005395
(87) International publication number: WO9105849

(56) References cited:
- EP-A- 0 239 272
- FR-A- 2 564 854
- GB-A- 2 118 572
- US-A- 3 955 317
- US-A- 4 320 594
- US-A- 4 473 970
- US-A- 4 496 443
- US-A- 4 868 123
- WATER RESEARCH, vol. 13, 1979; GOLDMAN, pp. 1-19
- AUSTRALIAN JOURNAL OF BIOTECHNOLOGY, vol. 1, no. 1, June 1987; "Wallace enter algal Synthesis with Biotechna", .... (unreadable text) 3 pages, see entire document
- WORLD PATENTS INDEX LATEST, section CH, week 8339, class D, AN=83-775791, Derwent Publications Ltd, London (GB)

## Description

### BACKGROUND OF THE INVENTION

### 1. Field of the Invention

This invention relates to a computerized process and modular apparatus for continuously growing photosynthetic microbes in liquid suspension, including a process and apparatus for harvesting the microbes so produced.

### 2. Description of Related Art:

The production of photosynthetic microbes, in particular unicellular algae and some bacteria, has long been recognized as having vast commercial potential in the production of useful products such as bulk chemicals, pharmaceuticals, and food for all commercially important aquatic animals (e.g. oysters, shrimp, and fish). Furthermore, photosynthetic microorganisms are capable of removing and transforming noxious compounds from their environment, including carbon dioxide-, inorganic salts of nitrogen and phosphorous, heavy metals, and a variety of toxic organic compounds.

The obvious utility of mass production of photosynthetic microbes resides in the process of photosynthesis itself. Given the appropriate supply of light, water, and carbon dioxide (CO₂), photosynthetic microbes can utilize sources of essential nutrients such as nitrogen (N) and phosphorous (P) to convert solar energy into chemical energy. Thus, the process of growing or culturing photosynthetic microbes involves (1) the introduction of nutritionally complete medium to a contained volume of culture, (2) maintenance of optimal growth conditions in that volume, and (3) subsequent harvest or removal of the microbial cells from the spent medium. All culture programs must devise methods to accomplish each of these processes efficiently.

It is yet to be established that mass culture systems found in the prior art can be both reliable and economical. Production figures serve to put this problem in perspective. In the laboratory, under highly controlled conditions and in small volumes (of the order one liter), yields in excess of 75 g dry weight/m²/day have been achieved (Thomas et al., Solar Energy Research Institute Report #CP-231-2341, Aquatic Species Program Review, "Cultural requirements, yields, and light utilization efficiency," 1984, pp. 7- 63). In natural environments, however, production under unusually favorable conditions is no higher than 15-20 g dry weight/m²/day (Parsons et al., Biological Oceanographic Processes, 3rd edition, Pergamon Press, NY, 330 pp.). Attempts at mass culture have generally been no more produdtive than culture in natural environments - attaining productivity of 20-25 g dry weight/m²/day (Goldman, Water Res., "Outdoor algal mass cultures - I. Applications", 1979, 13:1-19; Laws et al, Biotech. Bioengr., "A simple algal production system designed to utilize the flashing light effect", 1983, 25:2319-2335; Weissman et al., Solar Energy Research Institute Report #SP-231-3206, Aquatic Species Program Review, "Design and operation of an outdoor microalgae test facility", 1987, pp. 231-252).

Culturing of microbes in open channels or ponds has been practiced in many locations. The obvious drawback of this method is the potential for contamination from both biogenic and abiogenic airborne particulates, all of which detract from the purity of the culture. Contamination by hostile microbes is a primary threat which can in practice be overcome for only a handful of cultured species which are able to withstand extraordinary conditions of salinity or pH.

Open cultures have also suffered from a lack of control, such as inadequate control of physical and chemical conditions (e.g. turbulence, nutrient concentrations, dissolved gases) which will determine optimal growth of the microbial culture. Because of their large size, open systems are "batch" cultures, which grow from low to high cell concentrations with concomitant changes in physiological state and biochemical composition.

Finally, open culture systems are not economical to construct. Large open pond systems may require expensive laser-levelling, large berms or dikes, and extensive use of plastic lining materials. Construction economics are prohibitive in regions of highly variable topography or hard substrate. Open raceway systems may be more adaptable to variable topography, but the rigid materials, which both comprise and support the raceway system (e.g. U.S. Patent 4,320,594), are highly expensive. In general, then, open systems have been neither reliable nor economical.

More elaborate approaches have involved the use of horizontally placed flexible tubing to contain the culture medium (e.g. U.S. Patents 2,732,663, 3,955,317, and 4,473,970; Italian Patent Application No. 21522 A/78). The problems associated with such systems have included: (1) low velocity of flow through the system which allows settling of microbes on the bottom of the tubes; (2) inability to control temperature or, when temperature control was attempted by floating the system in water (U.S. Patent 3,955,317), the difficulty in locating and repairing potential leaks in the system and the potential for contamination through such leaks; and (3) lack of efficient, automated and inexpensive control systems, which both monitor and control conditions within the culture.

An alternative method has recently been proposed in U.K. Patent No. 2,118,572, wherein flat panels of about one square meter, containing small diameter tubing wound against the panel surface, are placed vertically. Problems associated with such a system include: (1) the instability of panels under adverse weather conditions; (2) high land usage resulting from shadows cast by the vertical panel as well as the inability of the panel to support a large volume of microbial culture; and (3) higher energy costs of pumping culture medium upwards against gravity.

A more recent method suggested by European Patent Application EP-A-0 239 272 involves a small diameter tube wound upwards in a coiling fashion upon a cylindrical support structure of approximately 2 meters diameter. In the 8 meter tall cylinder example given, the total culture volume appears to be about 1,269 liters, which would only amount to a culture concentration of 404 liters per square meter.

The disadvantages of such a system include: (1) high cost of the elaborate support structure; (2) high cost of the tubing (the example in the patent application requires 1,347 meters of tubing with a total surface area of 126 square meters, or 0.099 square meters of tubing per liter of culture); (3) high land usage and resultant cost, because the shadows cast by each vertical cylinder require the cylindrical units to be widely spaced (the example in the patent application suggests a spacing of 4 meters between cylinders, thus bringing the effectively occupied area of each cylinder to 16 square meters for 1,269 liters of culture, for a final culture concentration of 79.3 liters per square meter); and (4) high energy costs of pumping the culture medium to overcome high head pressure due to (a) 8 meters of static head and (b) large frictional losses in the small diameter tubing.

An important additional problem which has existed with all systems is the difficulty experienced in reinoculating systems where the culture has become contaminated or has otherwise "crashed" or become unproductive. In the operation of large-scale open systems, the process of resuming productive capacity generally requires three to four weeks. The containment device must be emptied, thoroughly cleaned, reinoculated, and then allowed to regain high cell density.

The single most important indicator of successful operation of the described system is productivity. It will be appreciated that all presently used methods of measuring productivity require that a sample of culture be physically removed and experimentally manipulated. This is true whether one employs gravimetric measurements of samples taken at certain time intervals, or whether one uses more sophisticated radiotracer techniques, such as measuring the rate of incorporation of ¹⁴C-bicarbonate into cells.

It will further be appreciated that the ability to control the dilution rate of a culture not only allows for control of the growth rate, but may also be used to determine the biochemical characteristics of the microbes grown therein. For example, it is well known that many microalgae produce predominantly proteins when in exponential growth phase, and predominantly fats in denser cultures associated with stationary growth phase. What is needed is the ability to control dilution continuously.

In summary, prior systems which are simple and relatively economical have proven to be unreliable; whereas, those which are more complex and provide a greater measure of reliability, have proven to be uneconomical. This economic barrier to reliability is clearly demonstrated by patterns of commercial usage. In large-scale commercial production of photosynthetic microbes, it is the simple and relatively economical systems that are used almost exclusively. The unreliability of these systems is clearly demonstrated by the fact that fewer than a dozen species of commercially valuable photosynthetic microbes are produced in such systems. There are tens of thousands of species which cannot reliably be cultured in open systems.

None of the prior art systems combine both (1) the ability to reliably produce photosynthetic microbes by automatic control and optimization of conditions in the culture, and (2) sufficiently economical means in order to justify production on commercial scale.

### SUMMARY OF THE INVENTION

The general objective of this invention is to provide an efficient, reliable, easy to use and maintain, large-scale system for culturing a variety of photosynthetic microorganisms, which maximises the yield and greatly reduces the potential for contamination by biogenic or biogenic particles.

To achieve this objective, this invention in one aspect comprises a method for culturing photosynthetic microbes and cells of photosynthetic macrophytes in a closed, continuous, circulating system, comprising:
a) pretreating source water to be used as culture medium;
b) circulating said pretreated culture water through a closed, continuous, conduit which is substantially horizontally disposed and the walls of which are transparent to sunlight within a protective growth module which is also transparent to sunlight;
c) inoculating said circulating culture water with cells of a photosynthetic species;
d) producing a substantially continuous flow regime having a Reynolds Number of at least 2000 in said circulating culture water ;
e) monitoring turbulence and one or more physical, chemical or biological properties of said circulating culture water, containing photosynthetic cells within said closed conduit;
f) automatically comparing the value of said observed physical, chemical or biological properties of said circulating culture water to a predetermined optimum value for said property;
g) automatically adjusting said physical, chemical or biological property of said circulating culture water, when said observed value deviates from said optimum value;
h) isolating a concentrated portion of photosynthetic cells grown in said conduit from said circulating culture water without affecting total volume; and
i) separating and recovering said photosynthetic cells from said circulating culture water.

In another aspect, the invention comprises apparatus for large-scale culturing of photosynthetic cells of a desired species, including microbes and cells of photosynthetic macrophytes, in a closed, continuous, circulating system, comprising:
a water pumping and filtration system supplying pretreated culture water for growing a culture of photosynthetic cells;
at least one growth module containing a continuous, closed culture conduit substantially horizontally disposed for circulating culture water, the walls of said conduit being transparent to light, the conduit being disposed within a protective growth module which is transparent to sunlight;
means for circulating said culture water through said closed culture conduit;
means for inoculating said circulating culture water with a cell mass of said photosynthetic cell species to form a concentration thereof within said culture water;
means for monitoring turbulence and one other physical, chemical or biological property of said culture water circulating within said closed culture conduit, and comparing the observed values of said monitored properties of said circulating culture water to a predetermined optimum value for each said property;
means for producing a substantially continuous flow regime having a Reynolds Number of at least 2000 in said circulating culture water;
means responsive to said comparison of said observed and optimum turbulence values for changing the Reynolds Number of said turbulent flow regime;
output means for isolating a portion of said circulating culture water containing said cell mass from said conduit without affecting the level of culture-containing circulating water;
input means for introducing and maintaining an appropriate level of said culture water into said conduit; and
means for separating said photosynthetic cells from said circulating culture water isolated by said output means.

A further object of the invention is to provide a closed culture system, wherein the critical components of containment, structural support, monitoring and control, as well as methods of installation and operation are greatly improved, simplified, and economical.

A still further object of the invention is to provide a method enabling a direct, automatic and immediate measurement of productivity accomplished in situ requiring no physical removal of samples from the culture medium.

A still further object of the invention is to provide the ability to control biochemical characteristics, such as the types of molecules produced by photosynthetic microbes, by allowing continuous control of dilution, and hence the cell concentration within the growth medium.

Still further objects and advantages of the invention will become apparent to those skilled in the art upon reading the entire disclosure contained herein.

### BRIEF DESCRIPTION OF THE DRAWINGS

Figure 1 is a flow diagram showing the basic processes involved in the operation of the microbial culture system of the invention.

Figure 2 is a plan view of a microbial culture facility employing the system of the invention.

Figure 3 is a schematic diagram of a water pumping and pretreatment facility in accordance with the invention.

Figure 4 is a plan view of a photosynthetron unit employed in the system of the invention.

Figure 5 is a perspective view, partially cut away, of a growth module in accordance with the invention.

Figure 6 is a cross-sectional view of a growth chamber of the growth module of the invention taken on Line 6-6 of Figure 5.

Figure 7 is a plan view of the control components in the growth module of the invention.

Figure 8 is a cross-section of a monitor/control manifold of the invention taken on Line 8-8 of Figure 7.

Figure 9 is a cross-section of the harvesting facility for a photosynthetron unit of the invention.

Figure 10 is a cross-section of a sedimentation tank used in the system of the invention.

### DETAILED DESCRIPTION OF THE PREFERRED EMBODIMENT

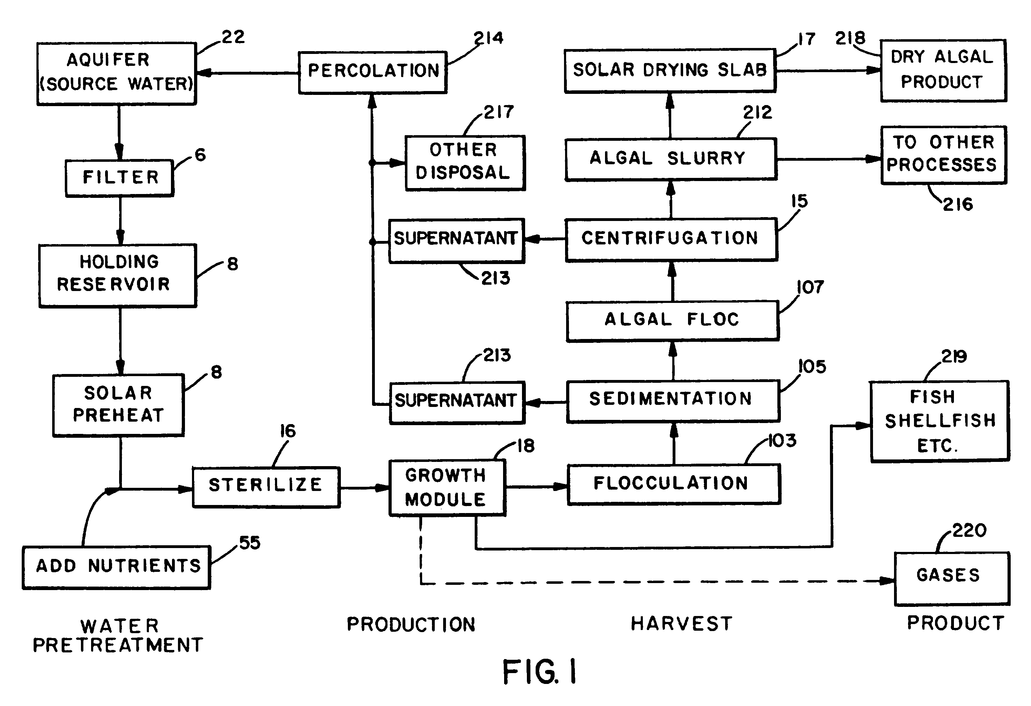

The basic principles and processes involved in the production of photosynthetic microbes in accordance with the invention are outlined in the flow diagram of Figure 1 and below. Operation of the system of the invention may be partitioned into three principal functions: (1) water pretreatment, (2) microbe production, and (3) harvesting. Figure 1 also shows that the system of the invention is capable of maximum conservation of water resources by recycling all water not subject to evaporation.

In the water pretreatment stage, water is pumped from an aquifer 22 or other source (fresh or marine), preferably passed through a filter 6, and then pumped into a large holding reservoir 8. The cover of the holding reservoir, preferably made from a dark plastic material, e.g., black polyethylene, prevents contamination of the prefiltered water, and promotes heating without exposing the prefiltered water to sunlight, which can encourage unwanted premature growth of photosynthetic microbes in the holding reservoir. Filtration may be accomplished by a variety of conventional techniques, such as rapid sandbed filtration for large quantities of water or diatomaceous earth for smaller quantities. Under certain circumstances, filtration may not be necessary, such as in the use of deep ocean water from oligotrophic regions or in the use of particulate-free water from certain aquifers. Obviously, if the water is already free of particulates, the filtration can be eliminated.

Preheating of the water is preferably accomplished by passive solar heating 8 in a covered holding reservoir. The preheating process may be necessary in the case of relatively cool source water, such as ground water which, if not geothermal, may be at a lower temperature than is required for optimal growth of many species of microbes (between about 24-35°C). Thus, preheating permits the temperature of the culture to be adjusted appropriately prior to introduction into the circulating growth system. Obviously, if the source water temperature is already at the appropriate culture temperature, the preheating step can be eliminated.

While solar heat is preferred for economic and environmental reasons, other sources of heat such as a conventional heater could be used to provide the necessary temperature adjustment.

A sterilization step 16 may be necessary in the case of source water expected to contain living microbes or potential predators, which might threaten the existence of the microbes being grown in the culture system. The preferred method of sterilization is by treatment of the medium with ultraviolet light, but it may also be accomplished by other conventional means. In many cases, filtration would be expected to eliminate living particles. Obviously, if analysis of the source water indicates no substantial contamination of the source water with unwanted microbes or potential predators, the sterilization step is not required.

Nutrients may be added to the water as it enters the growth module as at 55. In the production stage, water is distributed from the holding reservoir 8 through the sterilization processor 16 to individual growth modules 18. If the holding reservoir is maintained at a higher elevation than the growth modules, distribution from the holding reservoir can be accomplished under the force of gravity. Otherwise, a pump is required to feed the water into the growth module 18.

Optionally, the growth module may be provided with a means for collecting dissolved gases 220, such as oxygen, which are produced by the photosynthetic microbes grown therein. The growth module, which is described in greater detail hereinafter, may be characterized as a closed, controlled, continuous growth environment for photosynthetic microbes.

Harvesting is accomplished by a separation process, preferably a multi-stage process, with concentration of microbial biomass increasing at each step until a final dry product is achieved. There are, however, applications described below where a dry product is not required and the harvested culture in suspension is used directly as at 219.

The first step of harvesting is flocculation 103. Harvest water is pumped, or allowed to flow by gravity, from the growth module 18, flushing out the photosynthetic microbes grown therein. After leaving the growth module 18, the harvest water passes through an electroflocculator which creates large aggregates of microbes. Alternatively, flocculation 103 may be accomplished by the addition of chemical flocculants, such as alum, to the medium as it enters the sedimentation tanks 105. The aggregates produced by flocculation should, preferably, have sedimentation rates at least two orders of magnitude greater than those of the individual microbial cells of which they are composed.

Immediately after leaving the flocculator 103, the harvest water, containing the large aggregates of microbes, is passed into a series of sedimentation tanks 105. Sedimentation generally takes place in several hours, concentrating the algae by a factor of about 50 and yielding a concentrated algae floc 107 and a supernatant liquid.

The next of the harvesting procedure is centrifugation 15. Concentrated microbial floc 107 is removed from the sedimentation tanks 105 and passed into a high volume, continuous centrifuge 15 in which is it centrifuged, normally at 3,000 to 30,000 rpm to produce a supernatant 213 and a concentrated algal slug 212 having 70% to 90% water.

Supernatant 213 combined from both the sedimentation tanks 105 and the centrifuge 15 is passed preferably underground, to a percolation field 214 where it is recycled into the aquifer 22. Percolation underground or otherwise in the absence of light serves to kill any photosynthetic microbes which remain in the supernatant, so that they are not introduced into the aquifer 22. Natural percolation processes also serve to strip the medium of most dissolved organic and inorganic materials, thus providing generally pure recycled water to the aquifer 22.

The high speed continuous centrifugation 15 provides yet a further dewatering step, concentrating the microbial biomass by a factor of 20-50. In the situation where the growth modules 18 are used to remove contaminants or other elements or compounds from the water, the supernatant is maintained separately from the source water as at 217 and not returned to the aquifer 22.

An optional additional harvesting step employs a drying slab 17. Microbial concentrate from the centrifuge is distributed in a thin layer atop a concrete slab. Drying of the slurry concentrate 212 is accomplished by evaporation. Maximum evaporation rates are maintained by covering the drying slab 17 and automatically controlling the relative humidity and temperature within the covered slab housing.

In the case where microbes are not required to be harvested as a dried product 218, for example, when they are provided as food for fish, shellfish, or other economically valuable consumers 219, the harvesting step may be eliminated. In such cases, output harvest from the growth modules 18 may be pumped or passively moved by gravity directly into such containment systems as might be provided for the culture of the fish, shellfish, or other consumers 219. Optionally, a feedback system may be provided, whereby harvest water containing photosynthetic microbes is distributed on demand to the systems containing microbial consumers.

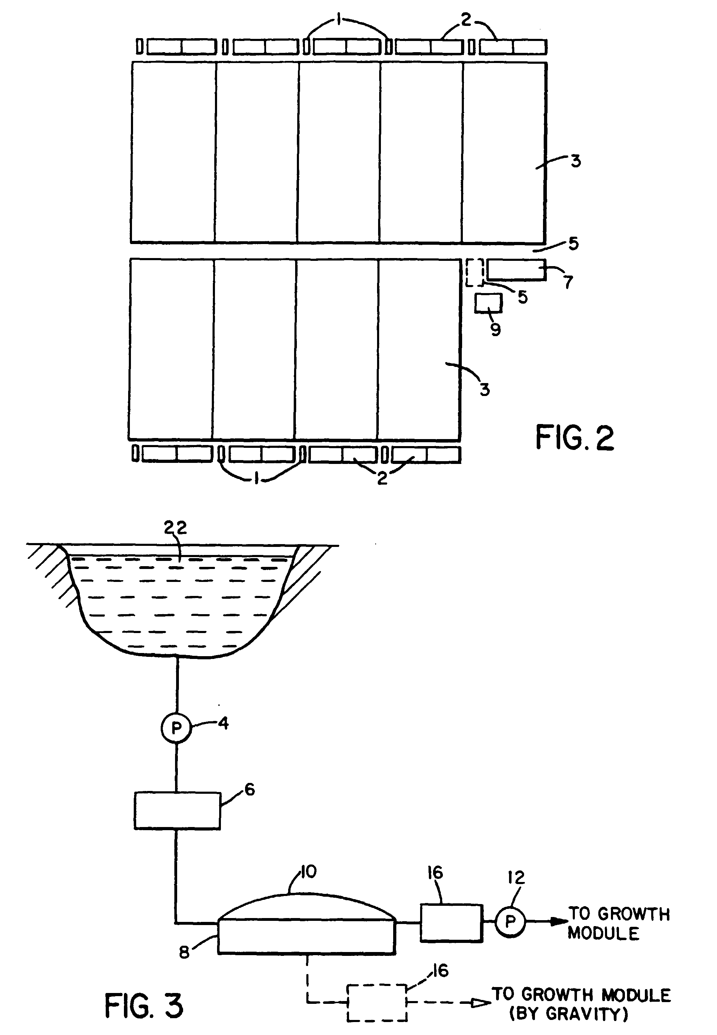

Figure 2 shows a plan view of a typical facility for the culture of photosynthetic microbes employing the system of the invention. Regardless of its actual size, the facility may incorporate certain basic features, including a water pumping and filtration system 1, a solar preheating and sterilization system 2, and one or more "photosynthetron" units 3, which are comprised of one or more growth modules and harvesting means. Roads and pathways 5 provide easy access to all parts of the facility. A central warehouse 7 and an office and data processing system 9 are commonly also part of the facility.

The purpose of the water pumping and filtration system 1 and the solar preheating and sterilization systems 2 is to bring water from the aquifer or other water source; filter out biotic or abiotic particulates; preheat the water, if necessary, to approximate required culture temperature; remove living microbes which may have survived the filtration process; and to deliver preheated sterile water to the photosynthetron 3.

Figure 3 shows a schematic diagram of the water pumping and filtration system of the invention. Source water from the aquifer 22 is transported by a pump 4 through a filtration unit 6 to a holding reservoir 8. The holding reservoir has a dark cover 10, which helps to prevent contamination of the water and promotes solar preheating. Preferably a light opaque plastic, the cover is constructed in such a manner that an air space is maintained between it and the water surface, and it faces the atmosphere with a convex surface, whose shape is maintained either structurally or by means of positive air pressure.

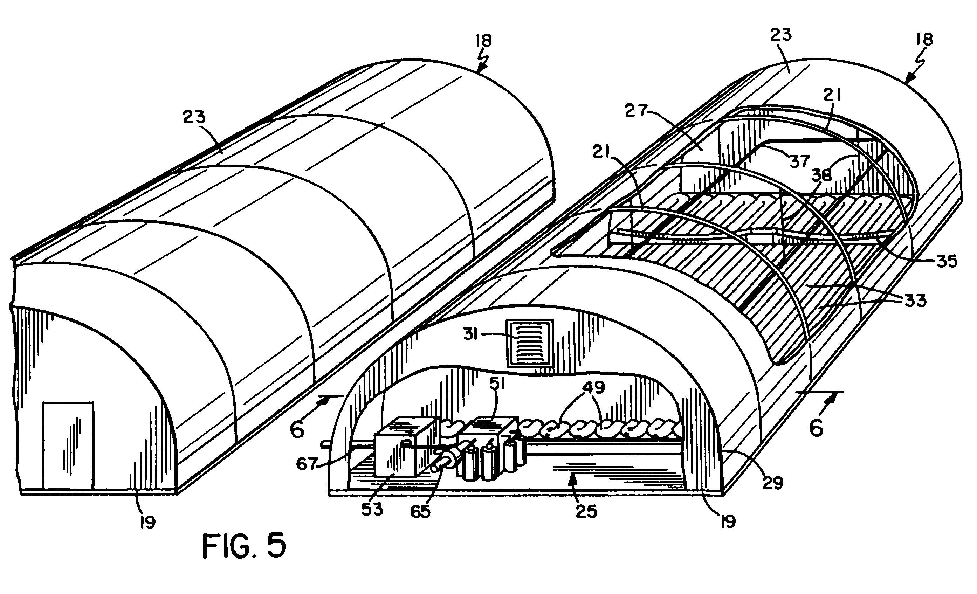

From the holding reservoir, the water is distributed through a sterilizer 16 to individual growth modules (Figure 5). If the reservoir is at a higher elevation than the modules, distribution is accomplished by gravity. Otherwise a pump 12 is required.

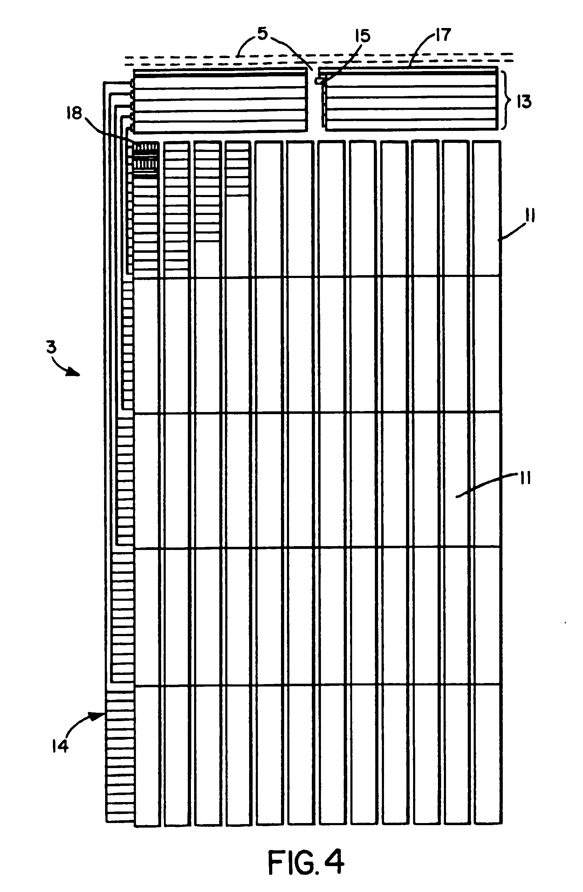

A photosynthetron unit 3, shown schematically in Figure 4, is a single self-contained unit of the system of the invention. It possesses all of the necessary equipment to produce and harvest microbial biomass. The bulk of its area is covered by arrays 11 of growth modules 18. The remaining area of the photosynthetron unit is comprised of harvest delivery systems 14, sedimentation tank arrays 13, a continuous centrifuge 15, and drying slab arrays 17. While only five harvest delivery systems for five growth module arrays are shown in Figure 4, it should be understood that any number may be used and that there will be a separate system provided for each array. Each growth module 18 in a photosynthetron unit is connected to a water pretreatment reservoir, as previously described.

The growth module 18 is the basic production unit in the system of the invention. The module contains means for monitoring and controlling the important culture parameters, e.g. temperature, pH, carbon dioxide and growth rate. The culture contained within a growth module is closed, in the sense that it is not exposed to the atmosphere and also because it is in a closed fluid circuit.

Figure 5 provides an overhead view of a growth module. The housing for the module is formed by a rectangular floor 19, upon which is erected a hemi-elliptical frame shell 21 which supports a clear cover of polyethylene 23 or other suitable material. In cooler climates the floor 19 will preferably be constructed of concrete with an insulating subflooring (not shown), so as to prevent significant heat loss to the ground. The thickness of both the concrete floor and the insulating subfloor will depend upon the ambient external temperatures expected. In temperate climates, for example, the floor and subfloor each would be between 5-10 cm thick. It will be appreciated that in moderate climates, where ambient temperatures approximate those required for the photosynthetic microbes to be grown, the subfloor may be dispensed with altogether.

The floor 19, whether concrete, earth, or other material, is treated to provide maximum reflectivity of light toward the culture, which is contained in transparent tubing 33 overlying the floor. An effective and economical means of providing such reflectivity is through the use of white paint on concrete. In the case of an earthen floor, reflectivity may be provided by white sheeting material, which is permeable in order to provide drainage for water accumulated via condensation or from potential leaks in the system.

The inside of the module is partitioned into two sections; a monitor/control section 25 and a growth chamber 27. Both sections are entirely enclosed within the same environment. It is in the growth chamber that the growth of the culture of photosynthetic microbes takes place, while equipment for monitoring and contro. ing the culture parameters is located in the monitor/control section.

The end walls of the module are supported by an end frame 29. A fan 31 is positioned in the uppermost portion of one end wall. Automatic louvers (not shown) are positioned in the same location in the opposite end wall. The fan and louvers provide rapid air exchange for temperature control of the module environment.

Growth tubes 33 are disposed side by side on the flooring. An optional moving catwalk 35 traverses the module above the growth tubes to provide easy access to any location within the growth chamber. A sprinkler system 37, suspended from the ceiling of the growth chamber by supports 38, provides secondary temperature control for the chamber.

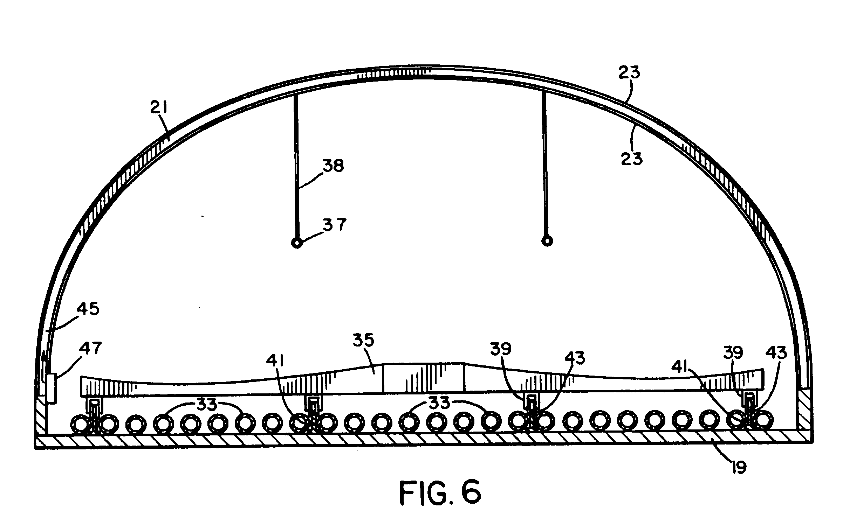

Figure 6 shows a cross-section of the same growth module taken across the growth chamber 27. The movable catwalk 35 is seen as raised above the growth tubes 33 by supports 39 equipped with wheels 41 and rails 43 which permit the catwalk to be moved in a direction parallel to the lengths of the growth tubes. It will be appreciated that the need for the catwalk depends to a large extent on the nature of the material used to construct the growth tubes. It is advisable where the tubes are made from thinwalled glass or other fragile material, but it is optional where the tubes are made from more robust or flexible plastic material. When tubing material is of flexible plastic material, access to all areas of the growth chamber may be achieved by gently pushing apart the tubes with one's feet and walking in the space so provided.

The polyethylene or other suitable external cover of the growth module may similarly be varied in construction according to prevailing climatic conditions. Figure 6 shows the preferred hemi-elliptical shell design useful in cooler climates. Here, two layers of clear polyethylene sheeting are separated by preformed aluminum or steel tubular supports to provide an area of insulating dead air space 45. The space is maintained by positive air pressure provided by a low energy air pump 47, which in turn provides further insulation. In more moderate climates, only a single layer cover is required.

The growth tubes 33 are preferably made of polyethylene, although any rigid or flexible material not harmful to plant tissue can be used providing it is substantially insoluble and impermeable to water, and transparent to visible light, but impervious to near-visible ultraviolet light. While low density polyethylene is most preferred for this purpose (particularly if it is of minimum thickness so as to reduce both light attenuation and cost), other possible materials include polypropylene, polyacrylate, polyamide, polycarbonate, water insoluble cellulose ester and polyester films, or glass.

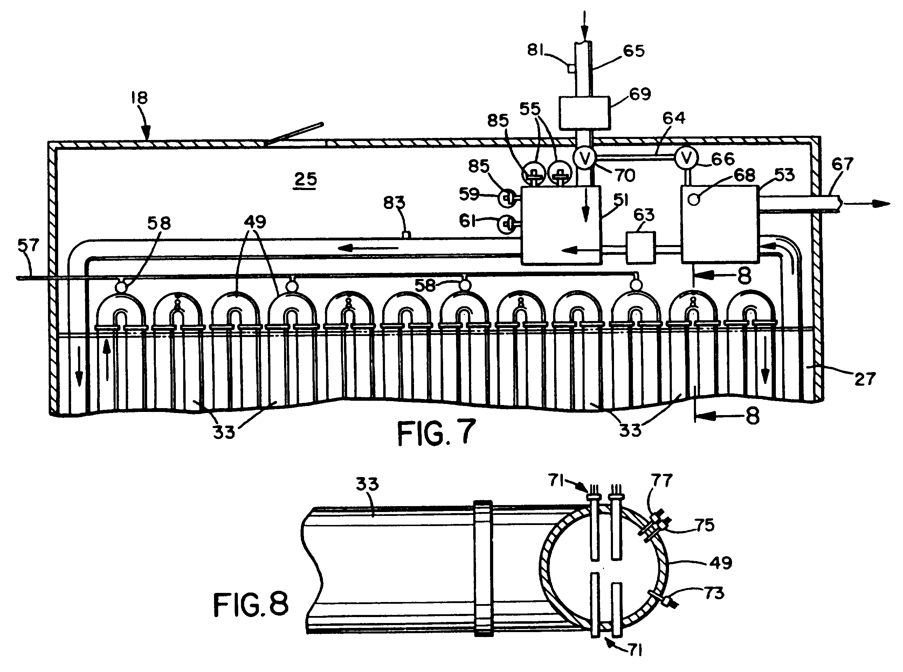

The floor of the growth chamber is covered by a plurality of growth tubes disposed horizontally side by side. At each end of the growth chamber, the growth tubes are joined by rigid 180° U-shaped returns 49 (Figure 7), preferably made of the same material. When both tubing and returns are made of polyethylene, they may easily be joined and sealed in seconds by application of heat in a process which forms a durable bond. The method of heat-sealing tubes and returns of like material provides for significant savings of both parts (which might otherwise be required for a mechanical seal) and labor.

The monitor/control section of the growth module is shown in Figure 7. This section contains two mixing tanks 51 and 53. Makeup water is introduced through an inlet 65 and passed through optional sterilization device 69. It will be appreciated that the need for sterilization will depend on the nature of the makeup water. The methods employed for sterilization may include ultrafiltration or exposure to gamma radiation.

Introduction and mixing of the inoculum of photosynthetic microbes, nutrients from tanks 55, and acid and base for pH control from tanks 59 and 61, respectively, take place in a mixing tank 51. Circulation of water in the system is accomplished by a pump 63 interposed between the two mixing tanks. Harvest water is released through an outlet 67 after entering tank 53. Harvesting is accomplished entirely by the force of gravity, since the base level of the outlet 67 coincides with the level of fluid in the system. Thus, when an inlet valve 70 is opened to allow makeup water through inlet 65 and into the mixing tank 51, the level of fluid increases, pushing harvest water out through the outlet 67.

During circulation, when the inlet valve 70 is closed, the pump 63 operates to keep the culture circulating in the growth module. The reservoirs provided by the tanks 51 and 53, in addition to providing the means for introducing and mixing necessary components in the system, maintain a static head which buffers against fluctuation in flow throughout the system.

The growth module operates in two modes, a circulating growth mode and a harvesting/refill mode. During both of these operational modes, flow characteristics are kept constant.

### Circulating Growth Mode of Operation

Flow rates are maintained by the pump 63, which operates to provide a turbulent flow for the circulating culture, preferably at Reynolds numbers exceeding 2,000. Turbulent flow is necessary to keep cells of certain photosynthetic microbes from settling out of the circulating water. This occurs particularly if they are not flagellated and thus cannot provide their own locomotion. Turbulent flow also insures that the cells in the growth medium are exposed to light which, in a dense culture, would be expected to be almost entirely extinguished at a depth equal to approximately 10% of the depth of the culture. The circulating/growth mode is allowed to continue for a time sufficient to allow the photosynthetic microbes to grow to a predetermined optimal biomass before harvesting.

During operation of the system, the physical, chemical and biological characteristics of the growth medium are continuously monitored at several monitor manifold locations 49. Monitored are pH, CO₂ content, temperature, optical density and other variables. Each of these variables is monitored from probes in the monitor manifold, as shown in Figure 8.

It can be seen that each manifold 49 is provided with optical probes 71, a thermistor 73, pH probe 75 and CO₂ sensor 77. Analog signals from the various probes are transmitted to a programmed microchip 79 (Figure 7) mounted on the exterior of the monitor manifold. At the microchip, these signals are digitized, interpreted and recorded enabling activation of control systems to maintain optimal conditions within the module and the growth tubes.

It will be appreciated that microprocessor control of the type described herein enables both monitoring and control of the culture in the growth module from a remote location, accomplished by standard telecommunication links. This presents a considerable advantage to a potential user who may wish to operate growth modules at multiple locations throughout the world from a single central location.

The pH of the system is monitored by probes which are present in each monitor manifold. While the particular optimum pH level will vary with the species of photosynthetic microbe being cultured; generally, pH levels between 6.5-9.5 are required for optimum growth. High pH may be downregulated by adding either CO₂ (see below) or by adding acid (preferably nitric acid), while low pH can be raised by adding base (preferably ammonium hydroxide). By selecting nitrogenous acid and base, pH regulation simultaneously adds nutrients to the medium. Depending on the nutritional requirements of the species being cultured, phosphorylated acids and bases may be used if phosphorous is preferred over nitrogen as the primary limiting nutrient.

The addition of acid and base takes place in tank 53 (Figure 7). Commands from the microprocessors activate a metering pump 85 on either the acid 59 or base 61 tank. The time interval between additions of acid or base will depend upon the residence time in the mixing tank, the circulation rate and the time lag of travel between successive monitoring manifolds. For a large scale system, a five minute interval is usually appropriate.

Software analysis of pH levels at the various manifolds demands that pH be regulated at two rates. Referring back to Figure 7, the first rate is that rate which is necessary to correct to ideal conditions, based upon monitoring values from the final monitoring manifold, i.e. the manifold closest to tank 53. The influence of this acid or base addition is checked at the first manifold, i.e. the manifold closest to mixing tank 51. If the pH value at this manifold has reached the desired level for ideal conditions, the first rate of acid or base addition continues for the remainder of the circulating/growth cycle.

A second rate of pH adjustment is determined by monitoring data from the manifold closest to mixing tank 51. If pH at this manifold has not attained the ideal level after allowing sufficient time for the added acid or base to reach that point, a second rate of addition is put into effect, which increases or decreases the rate of addition by a factor of 25%.

The monitoring and control of CO₂ is also mediated via the multiple microprocessors. An electrode in the monitoring manifold measures CO₂ concentration in the medium. The data is reported to the microprocessor, which compares the observed value to the predetermined optimum range for CO₂ concentration required to obtain optimum conditions for photosynthesis within the growth tubes. The optimal range will, again, be species dependent, but in all cases will be greater than 100 g/m³ of Carbon as either CO₂, carbonate or bicarbonate, and in no case will exceed the solubility of CO₂ in water.

Referring again to Figure 7, if the CO₂ concentration is observed to be below the optimum range, a solenoid valve 53 is opened to release CO₂ into control manifolds situated adjacent to and preceding each monitor manifold. As noted above, CO₂ may also be used to regulate pH. This is the preferred method of increasing acidity if CO₂ levels are low and nutrient concentrations are high, whereas the addition of acid is preferred when CO₂ levels are high and nutrient levels are low.

The biomass of photosynthetic microbes in the culture is determined by optical methods. In one method, measurements are made by reading optical density (Figure 8), but alternate optical methods may be appropriate, such as in vivo fluorescence of chlorophyll or other fluorescent pigments. Since optical density and pigment concentrations are proportional to the biomass of microbes, these methods provide a measure of the concentration of microbes present at each monitoring location within the culture. Furthermore, the configuration of the system allows for the direct measurement of productivity. Referring again to Figure 7, this is accomplished by taking into account the dilution of the culture with makeup water introduced through the inlet 65 and measured by a flowmeter 81; knowledge of the system flow rate measured by another flowmeter 83; and knowledge of the linear distances between monitoring manifold locations 49. As diluted medium flows through the system from one mixing tank 51 to another mixing tank 53, the concentration of microbes increases. Thus, the difference in cell concentration between two monitoring locations, separated by a known distance, which requires a known time to be traversed, is used by the analysis software to directly flocculate the amount of cell material produced in that period of time. Thus, productivity can be readily determined and improved or optimized by any user of this system.

It will be appreciated that, unlike any other system, the method by which this invention enables the measurement of productivity is direct, automatic, immediate, and, since it is accomplished in situ, requires no physical removal of samples from the culture medium.

Temperature control is also very important. All photosynthetic microbes grow optimally within rather narrow temperature ranges, with growth often decreasing significantly when temperature falls 2-3°C outside the optimal range. The fastest-growing species usually grow optimally in the range of 25-35°C. In general, growth rate increases slowly with increasing temperature, and then declines more rapidly at temperatures above optimum. In most cases, the problem of temperature control will be one of keeping temperatures in the growth module low rather than high. This is because the greenhouse-type design of the growth module can keep the temperature inside the module at approximately 20-25°C when the outside temperature approaches 0°C. With the addition of insulation, such as the double layer polyethylene cover shown in Figure 6, optimum temperature conditions can be obtained even in winter in harsh climates.

Keeping the temperature of the culture medium in the growth tubes from rising above the optimum level is accomplished by primary, secondary and tertiary cooling means. Referring to Figure 5, the primary cooling means comprises an exhaust fan 31 positioned at one end of the module. By selecting a fan of the appropriate size, the entire air mass of the growth chamber can be replaced by fresh air within a matter of minutes.

The primary cooling means is activated by any of the microprocessors in the monitoring manifolds, but preferably by the one farthest from the inlet 65 (the medium would be expected to have reached a higher temperature at that point than at any other monitoring manifold in the system) upon its receipt of a signal from a suitable temperature sensor in a monitoring manifold, indicating the temperature of the culture medium has exceeded a designated limit. If, in spite of the primary cooling, the temperature of the culture medium continues to rise, the secondary cooling means is activated.

Again referring to Figure 5, the secondary cooling means is comprised of sprinkler lines, which distribute a fine mist over the growth chamber, suspended over the growth tubes. Temperatures are lowered by evaporative cooling.

If the temperature continues to rise despite secondary cooling efforts, the tertiary cooling means can be employed. This means consists of introducing makeup water through the inlet 65, assuming that makeup water will be of a temperature significantly lower than that in the insulated growth chamber. This tertiary means of cooling will, of course, cause harvesting and will have the effect of diluting the culture below cell concentrations stipulated for maximum production. While this results in less than optimal conditions, it is not expected to happen frequently because it will be used only as a last resort. Also, to lose a fraction of the culture is preferable to losing the entire culture due to overheating.

While Figure 8 illustrates only pH, CO₂, temperature and optical density probes, it is understood that numerous other variables could be monitored in the same manner. For example, electrochemical sensors for analyzing the concentration of inorganic and organic compounds may be added to monitor nutrients, products and/or contaminants which may be present in the culture. In addition, sensors for dissolved gases other than CO₂ may be included. For example, it may be desirable to monitor oxygen concentrations, since the rate of change of O₂ concentration is directly proportional to the rate of production of photosynthetic microbes within the culture. Light emitters and detectors, which monitor fluorescence of pigments contained in the microbes at preselected wavelengths, are useful to insure that the desired microbe species is being produced, since each species has pigments with well defined fluorescence characteristics.

As previously stated, the optimal ranges for the various process parameters, i.e., pH, CO₂ concentration, temperature, etc., are species dependent. The particular parameters for individual species of photosynthetic microbes are well known or readily ascertainable by those skilled in the art.

The growth module is designed to conserve heat. The thermal mass of the floor, if manufactured from concrete, is approximately one-third that of the culture medium. Thus, at night as ambient temperatures decrease, the floor will act as a heat source.

Under certain conditions, the fan system can also be utilized to increase temperature in the growth chamber. When the outside air is warmer than the air inside the module, generally in the morning, the exhaust fan can be turned on to bring warm air into the growth chamber. Conversely, if the module is to be operated in an environment which is generally quite warm relative to the requirements of the microbes to be cultured, then it may be preferable, as discussed earlier, to have the floor act as a heat sink, rather than as a source. This would be accomplished by dispensing with the use of the concrete floor and using only leveled earth as flooring.

### Harvest/Refill Mode of Operation

The second operational mode of the growth module is the harvest/refill mode. This mode is comprised of four basic steps, acting simultaneously: (1) diverting a portion of the circulating medium to output (harvest); (2) adding makeup water (refill); (3) adding nutrients; and (4) conditioning the medium. Referring to Figure 7, in order to harvest, a portion of the circulating medium is diverted to the outlet 67 by adding makeup water into mixing tank 51. This addition of new water to the system will cause the level of the system to rise above the level normally maintained during the circulating growth mode, causing culture medium to spill over through outlet 67 located in mixing tank 53, farthest from the inlet of makeup water in terms of conduit. As previously stated, this method of harvesting by gravity is accomplished because the height of the outlet 67 actually defines the operating level of the system during the circulating growth mode.

This process embodies three principal advantages over other photobioreactors. First, by using gravity rather than pumps or other mechanical devices to effectuate harvest, there is increased reliability, since gravity always works even when mechanical devices may not. Second, the cost of harvesting is considerably reduced. Third, the procedure of harvesting at the effective end of the tube culture, i.e. from tank 53, results in harvesting of the portion of culture with the highest possible cell concentration. In this invention, the lowest concentration of cells is found in mixing tank 51 where makeup water is added. As the culture moves through the system toward tank 53 the cells continue to divide, reaching their greatest concentration at the point of harvest.

This harvesting method is not possible with typical laboratory bioreactors or with open ponds, because, in both cases, fresh makeup water is stirred into the culture, causing it to be homogeneous, and consequently, forcing harvest of a diluted culture.

The time to harvest may be predetermined as either a set time, or upon achieving a specific cell concentration in the culture. For example, if microbes are cultured for the purpose of directly feeding fish with nocturnal feeding habits, it would be preferable to harvest only at night. However, if microbes are being cultured for maximal production of biomass or desirable product, it would make sense to maintain the cell concentration at a level considered to be optimal for such production.

In either case, it is clear to those skilled in the art that the rate of dilution (which equals the rate of harvesting) will dictate the growth rate of microbes in the system, up to the limit of their capacity to reproduce. For example, if the culture is diluted at a rate equal to two full volumes of the system per day, the cells in culture will experience a growth rate of two doublings (divisions) per day.

In the preferred embodiment of the invention, fresh makeup water is added continuously; however, it may be added in batches if desired. Thus, during the harvest mode, two sources of water are entering mixing tank 51; the circulating water containing the microbial culture flowing from tank 53, and fresh makeup water coming from the water pretreatment unit. These two sources of water enter tank 51 and are mixed in the proportions necessary to dilute the culture to the appropriate concentration.

If the rate of dilution is to be determined by the culture concentration rather than by a predetermined time, signals from the optical density probes 71 (Figure 8) indicate whether the predetermined concentration has been reached. These signals are interpreted by the microprocessor, which may effectuate the addition of fresh makeup water by activating the variable-flow solenoid valve 70 and allowing makeup water to enter through inlet 65. The rate at which fresh water enters is determined by the degree to which the variable-flow solenoid valve 70 is opened. In one example of operation, the rate of addition of makeup water, measured at the flowmeter 81, will be set at equal to one-half the flow rate of recirculating water measured at another flowmeter 83. This rate will result in diluting the culture to a concentration just half of that which is being harvested from tank 53.

As previously stated, it will be appreciated that the ability to control the dilution rate of the culture not only permits control of the growth rate, but can also be used to determine the biochemical characteristics of the microbes grown therein. For example, it is well known that many microalgae produce predominantly proteins when in exponential growth phase, and predominantly fats in denser cultures associated with stationary growth phase. Thus, by allowing continuous control of dilution, and hence the cell concentration within the growth medium, the system of the invention embodies the ability to control biochemical characteristics of cells.

Nutrient addition is a simple exercise accomplished by activating the nutrient metering pumps 85 in nutrient tanks 55. The nutrient metering pumps dispense concentrated nutrients as necessary for the optimal growth of a particular microbial species being cultured. For example, superphosphate, urea, silicate, trace metals such as zinc, iron, vanadium, etc., vitamins, and other growth enhancing materials, are added from nutrient tanks 55 located adjacent to tank 51. These nutrients are concentrated by a factor of approximately 4,000 relative to the final concentration in the growth system.

The nutrients are commonly added to the fresh culture water medium in tank 51 at a rate of approximately 1/2 ml/sec for a three hour harvest/refill period. The rate of nutrient addition is adjusted to rapidly bring nutrient concentrations within the culture medium to predetermined optimal levels. These levels are selected on the basis that all nutrients will be consumed by the end of the growth mode. Alternatively, the addition of nutrients can be controlled by the microprocessors based upon readings by sensors placed in the monitoring manifolds, in an analogous manner to pH and CO₂ control, previously described. The nutrient tanks 55 contain sufficient nutrients for operating the growth module for several months.

Conditioning of the new medium also takes place in mixing tank 51. A different set of control parameters are used to adjust pH and CO₂ levels during the harvest/refill mode. Based upon the known pH and CO₂ levels in the fresh makeup water, CO₂ and acid or base are added at constant, predetermined rates during the harvest/refill mode. As described previously, acid and base additions are made in tank 51, whereas addition of CO₂ takes place at separate CO₂ control manifolds located at various points of the growth tube system. The rates of addition are such that fresh makeup water is brought up to optimal specifications for pH and CO₂ by the end of the harvest/refill cycle. Thus, closed-loop control of pH and CO₂ is suspended during the harvest/refill mode.

### Physical Parameters of the Growth Module

It will be appreciated that there are both upper and lower limits to the number of growth tubes which may be used in a typical growth module, although the processes and principles of operation are identical regardless of the number of tubes employed. The lower limit of two tubes is set by the minimum number which can conform to the configuration required to recirculate, monitor and control the culture as previously described in detail.

The upper limit of 32 tubes is governed by several factors, including the pressure capacity of the tubing, the amount of positive pressure provided by the pump used to recirculate the culture, and the amount of dynamic head generated by frictional losses accumulated during recirculation. Tubing material with a low pressure capacity, such as thin walled flexible polyethylene, would demand a smaller number of tubes in the system than tubing material with a high pressure capacity, such as thick walled glass or polyacrylate.

The greater the total length of tubing in the system, and, particularly, the more 180° returns there are in the system, the greater will be the frictional losses of pressure. The greater the frictional losses, the more pressure will be generated by the pump, and, consequently, the greater will be the risk of exceeding the pressure rating of the tubing material.

The system of the invention may embody a means to overcome frictional losses through the use of gravity. If, for example, frictional losses in a given configuration are calculated to generate a dynamic head equivalent to 15 cm, this could be overcome by building the floor of the growth module at an angle such that the tube containing the most dilute portion of the culture, i.e. closest to tank 51, is exactly 15 cm higher than the tube containing the most concentrated portion of the culture, i.e. the tube entering tank 53. The floor is graded on a linear incline from one side of the growth module to the other. This method of utilizing gravity to overcome potential dynamic head pressure will have the effect of reducing the energy requirements of the pump, and will also reduce the total pressure in the system, permitting a longer system of tubing than would otherwise be feasible.

### Inoculation of Microorganisms

When a growth module is first placed in production, it is necessary to inoculate it with the desired species of photosynthetic microbe. There are two basic methods by which this is accomplished; inoculation from a laboratory culture, and inoculation from another growth module. Inoculation from a laboratory culture entails gradually increasing the volume of the system after addition of the inoculum.

Referring to Figure 7, the system is first temporarily replumbed so that the outlet from mixing tank 51 is joined to the second tube most adjacent to mixing tank 53, thus forming a closed loop comprising tank 53, pump 63, tank 51, and only two growth tubes. The replumbing is rapidly effectuated by the same method as described previously and used to connect growth tubes to the rigid polyethylene 180° returns, i.e. by heat fusion.

With the system now limited to two growth tubes, fresh makeup water is poured into tank 53 through a diversion connector 64 by opening a valve 66. Only enough makeup water is added to fill a small portion of the growth tube which is attached to tank 53. The water is kept in a restricted portion of the tube by placing an object beneath it such that the height of the tube at that point is raised above the water level in the tube.

The makeup water is now conditioned with nutrients and other desired additives by introducing these through a port 68 located in the upper surface of tank 53. The inoculum is then introduced through the same port, and the port is closed. As an example, one might use 30 liters of culture for the inoculation, in which case the initial volume of makeup water added would be approximately 150 liters. Although the ratios of concentrated laboratory inoculum to fresh makeup water will be species dependent, and optimum ratios will be determined empirically, the ratio will generally be in the range of 5:1 to 10:1.

During the initial period of growth of inoculum in the two-tube system, which will generally last for only a few days, the system is increased in volume by continued addition of fresh makeup water through diversion connector 64, and nutrients are added manually through port 68. The culture of microorganisms is permitted to increase in concentration. When both growth tubes are full of medium, the pump 63 can then be used to recirculate the culture medium. From this point on, continued expansion of the system to full operating capacity is a simple matter of replication.

When the culture has reached the desired cell concentration in the two-tube system, the temporary connection from the outlet of tank 51 to the second tube is removed, a 180° U-shaped return is used to connect tube number 2 to tube number 3, and the temporary connector is moved to a new tube further along in the system. Fresh makeup water is now added through inlet 65, and conditioned for nutrient content and pH by the method described for operation of the full system, i.e. adding required amounts from nutrient tanks 55 and acid and base reservoirs 59 and 61.

This process is repeated, by growing to the desired concentration, reconnecting the temporary connector, and adding fresh makeup water, until the entire system is at full operating capacity.

An estimate of how much time this inoculation process requires, in going from a two-tube system to a system which at full capacity is comprised of thirty-two tubes, can be easily given. If the desired species being cultured has a growth rate of two doublings per day, it would take two days to reach full capacity. On the first day, the system would be expanded from two to eight tubes (two doublings), and on the second day from eight to thirty-two tubes (two doublings).

The second method of inoculation is by addition of inoculum from another module. This method of the invention highlights one of the key advantages of culturing in a modular format. As an example, let it be assumed that a plurality of modules are to be operated, but that only the first of these has been inoculated with the desired microorganism species.

Referring again to Figure 7, the preferred method of the system of the invention is to connect adjacent modules to one another, by attaching the outlet 67 of the first module to the inlet 65 of the second module, and so on. These attachments (not shown) are made in such a way that valves, which are interposed between the modules, allow a temporary diversion of the harvest water of one module to the makeup water inlet of another.

The inoculation is accomplished, for example, by transferring a volume of growing culture equivalent to 10-20% of the total volume from the first module into the second module. The temporary inoculation valves are then closed, and the first module is allowed to continue operating in the normal fashion. The second module is then brought up to full operating capacity by adding fresh makeup water through its inlet 65.

It will be appreciated that this method of inoculation allows for extraordinarily rapid expansion of the entire system to full operating capacity.

In the event of malfunction or contamination of the growth module, the pump 63 is turned off, stopping recirculation, and fresh makeup water is added through inlet 65, without adding nutrients or otherwise conditioning. This water then flushes the system until all culture medium containing the microbes has been removed. If the malfunction is one which does not require the removal of fresh makeup water, nutrients can be added and the system can be reinoculated from an adjacent module as described previously.

If, however, the malfunction does require that fresh makeup water be removed, the growth tubes are emptied by gravity by disconnecting the last tube from its point of connection to tank 53, and allowing water to flow out. The malfunction can then be repaired. If necessary, some or all of the growth tubes can be replaced. As an example, all growth tubes in a thirty-two tube system can be replaced in a matter of hours by a single individual. The growth module is put back into operation by inoculating by either one of the inoculation procedures described above.

If the malfunction is caused by the presence of a contaminant in the culture, the system is sterilized by pumping gaseous ethylene oxide into the system, which is now devoid of culture microorganisms, through the CO₂ input 57.

### Harvest Procedure

The harvest procedure, i.e. the procedure for recovering photosynthetic microbes from the output of the growth module, comprises, in most cases, three basic steps; (1) flocculation, followed by (2) sedimentation, and finally (3) passive solar drying. This entire procedure takes place in the harvest area for each photosynthetron unit. In general terms, the harvest process comprises concentrating the microbial biomass in a series of steps.

Water from the growth modules flows through a flocculator and into a sedimentation tank where the floc settles. The concentrated floc is then pumped into a continuous centrifuge, where it is de-watered. The resulting slurry is then spread onto a solar drying slab to complete the harvest process. Because the harvest procedure is dependent on the species being cultured, it may not be necessary to use any or all of the separation steps. For example, in the case of filamentous species, such as many blue-green algae, a rotary-drum filter may adequately replace both the flocculation and centrifugation steps.

As another example, when the modules are used to supply food to higher organisms such as invertebrates or fish, these organisms effectively accomplish the harvesting procedure themselves from the concentrated culture diverted to their feeding area, and there is no need for any final drying and mechanical harvesting method.

The harvest facility for each photosynthetron unit incorporates a plurality of sedimentation tanks. Each tank serves a plurality of growth modules, to which it is connected by inflow pipes. An inflow pipe can carry the output from several growth modules. As an example, each photosynthetron may incorporate a total of 20 sedimentation tanks, each tank serving 90 growth modules, connected by 6-inch diameter inflow pipes. Each 6-inch diameter inflow pipe collects output from 15 growth modules. Thus, in this example, a total of 6 inflow pipes are connected to each sedimentation tank.

The number of sedimentation tanks required for a system will depend upon the throughput volume of the system and the amount of culture harvested per day. In the previous example, if only 10 sedimentation tanks are required for each harvest, the system would accommodate two harvests per day since there are 20 sedimentation tanks.

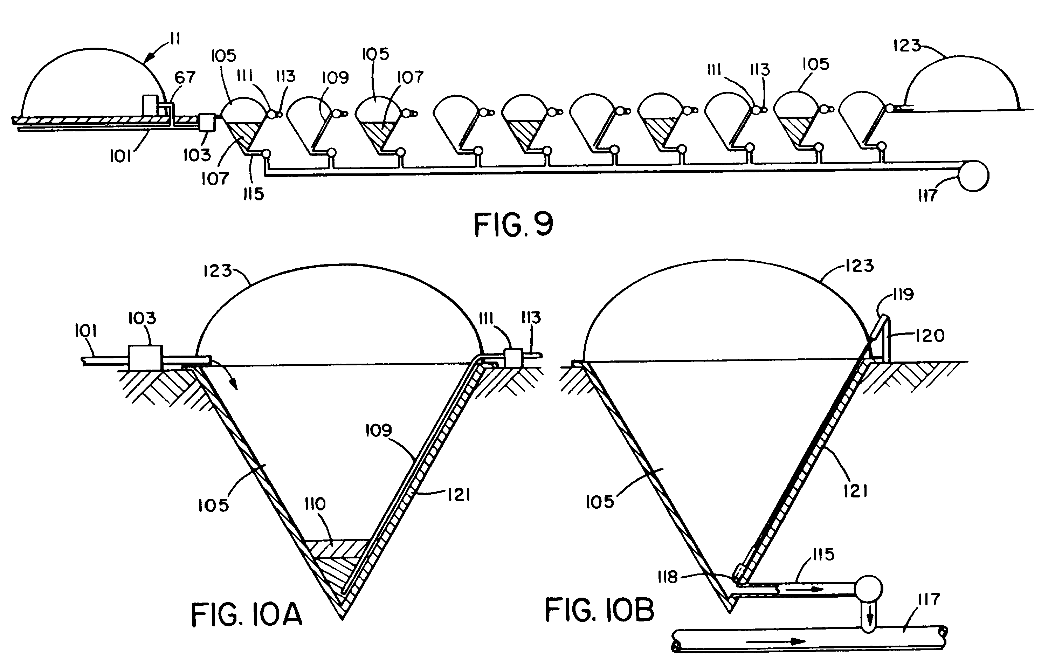

Figure 9 provides a cross-sectional view of the harvesting facility in a photosynthetron unit. Harvest output exits the growth module 11 through the outlet 67 and into the inflow pipe 101. The harvest is carried by the inflow pipe to a flocculator 103, where microbial floc is formed to facilitate sedimentation. The output of the flocculator 103 is connected to the input of a sedimentation tank 105, where microbial floc 107 is further concentrated by sedimentation.

The concentrated microbial floc is subsequently removed from the sedimentation tank through a drain pipe 109 by action of a pump 111. The output from all sedimentation tanks is then directed through an output line 113 to a central centrifuge (not shown), which serves all sedimentation tanks in the system. Each sedimentation tank also has a supernatant drain 115, which feeds into a main supernatant conduit 117, for recycling supernatant to the aquifer for reuse in the system or, in the case where the system is used for bio-filtration or to remove transparent purified water, to a new location.

From the central centrifuge, a microbial slurry is pumped to a drying slab 119 for the final step in the harvesting process. Figure 10 provides more detailed cross-sectional views of a sedimentation tank. In cross-section, the conical tank 105 appears triangular. The angle of incline of the tank wall is preferably 60°. While an angle of as low as 30° is sufficient for sedimentation of fine grain particles, the 60° angle is preferred since microbial floc is expected to settle less efficiently than fine grain material, such as sand.

The wall of the sedimentation tank 121 is preferably made of concrete. The tank is covered by dark covering material 123. The covering material insures that no water will be lost to the atmosphere through evaporation.

As shown in Figure 9, two drains service each sedimentation tank. The first drain 109 serves to pump sedimented floc 110 out from the bottom of the tank. The floc drain is a perforated pipe, which removes the concentrated with the aid of a pump 111, as output to the centrifuge. The second drain 115 in the sedimentation tank is a supernatant drain. Once the concentrated floc has been removed from the tank, a gate valve 118 is opened by a linear actuator 119 on a mount 120, causing supernatant water to flow through the supernatant drain 115, and into the main supernatant drain 117. The outflow of supernatant through the supernatant drain is governed by gravity alone, but could be effectuated by active pumping if water were to be returned to ground level or above.

Prior to entering the sedimentation tank, the harvest output from the growth modules is subjected to flocculation. Flocculation methods are well-known, and many techniques may be used, such as electroflocculation, or the use of inorganic flocculants such as alum, or organic flocculants such as chitosan.

As previously described with reference to Figures 9 and 10, the flocculated microbial output from the sedimentation tank is pumped to a central centrifuge. In most cases, a single centrifuge for each photosynthetron unit will be sufficient to sediment the combined floc output from all sedimentation tanks during a harvest cycle. A variety of continuous centrifuges are available for this application. It is preferable to use a plurality, e.g. two or three, of relatively low-capacity centrifuges rather than a single high-capacity centrifuge, so that a back-up is available in the event of equipment failure. With either single or multiple centrifuges, the most convenient place to locate the centrifuge facility is in a central location 15 as shown in Figure 4.

The microbial slurry produced by the centrifuge is pumped through conduits to the drying slabs 17 as shown in Figure 4. A drying slab may be as simple as a flat concrete slab, which is open to the atmosphere for evaporative drying of the slurry. Alternatively, the drying slabs may be constructed to have a module cover, exhaust fan, and an air entry way in the same fashion as a growth module. This construction has the advantage of protecting the drying algal biomass during foul weather and permitting control of the drying operation.

The provision for drying slabs is considered to be an optional step for use when a highly dry microbial biomass product is required. For many applications, the microbial biomass which exits the centrifuge and has a TSS between 15 and 20% will be sufficiently dry. In such applications, the drying slabs can be entirely eliminated from the system.

The harvesting procedure produces two sources of supernatant water, which is recycled either by percolating into the aquifer or into another water source for the facility. The first source of supernatant water is from the sedimentation tanks. The second is from the centrifuge facility. Both, the sedimentation tanks and the centrifuge are connected to a supernatant main, which carries the supernatant water underground to a percolating basin, where it may reenter the aquifer or be removed as purified water. One of the principal advantages of the system of the invention over conventional open air microbial bioreactor systems, is the tremendous reduction in evaporative losses. It is estimated that as much as 99% of daily water usage can be recycled to the aquifer, or released as purified water.

It will be appreciated that by virtue of the method of closed, continuous culturing embodied in the growth module of the invention, a variety of advantages are gained over conventional microbial bioreactor systems. As an example, the system of the invention may be applied to the production of molecular oxygen, the principal gaseous byproduct of photosynthesis. In conventional open pond culture systems molecular oxygen is lost to the atmosphere, but in the system of the invention, virtually pure molecular oxygen may be collected directly via tubes (not shown) emanating from various points along the length of the growth tubes.

As another example, the continuous culture method of the invention makes it possible to produce cells of consistent biochemical composition, which is a clear advantage when these cells are to be used as a source of chemicals or as food for invertebrates or fish with specific nutritional requirements. In conventional batch culture systems, it may be possible to produce cells of consistent biochemical quality, but these cannot be produced continuously.

As another example, the growth module of the system of the invention provides the ability to rapidly inoculate or reinoculate the culture of photosynthetic microbes in one growth module by using as an inoculum the harvest output from another growth module, which is at full operating capacity. This method of the invention, which derives from its modular nature, overcomes one of the major difficulties heretofore experienced in the operation of conventional culture systems.

As another example, the growth module of the system of the invention has, by virtue of its design, eliminated many of the disadvantages of open culture systems, e.g. contamination potential and lack of environmental control. At the same time, however, the growth module of the system of the invention provides for optimal growth of photosynthetic microbes, using methods and materials which are so efficient, such as the use of gravitational forces wherever possible and the use of dedicated microprocessors for monitoring and control of environmental variables, that closed culture as a commercial practice is for the first time rendered economical.

As another example, the method of operating the growth module of the system of the invention makes it possible to directly measure productivity of the photosynthetic microbes contained therein by methods which are automatic and economical. This is accomplished by combining the automatic measurements of cell concentrations at various points along the length of the system with measurements of the amount of time required to transit from one point to another.

### EXAMPLE:

The following tables provide design specifications and operating parameters of a commercial facility employing the system of the invention, capable of producing 5,555 metric tones per year of photosynthetic microbial biomass in one photosynthetron unit.

**TABLE I**

| DESIGN SPECIFICATIONS FOR THE PHOTOSYNTHETRON A 42.2-HECTARE, SELF-CONTAINED FACILITY. | | |
|---|---|---|
| I. | STATIC | Dimensions (MKS) |
| | Length | 1100 m |
| | Width | 384 m |
| | Plan area | 42.2 ha |
| | No. of growth modules | 1080 |
| | Total fluid volume | 40,600 m³ |
| | No. of sediment tanks | 20 |
| | No. of electroflocculators | 60 |
| | No. of centrifuges | 3 |
| | No. of drying slabs | 12 |
| | No. of preheating reservoirs | 1 |
| | No. of well pumps | 1 |

| II. | HYDRODYNAMIC | |
|---|---|---|
| | Water Input | 20,333 m³/day |
| | Water output | 20,255 m³/day |
| | Evaporation* | 78 m³/day |
| | Harvests per day | 1 |
| assumes 20% TSS at final dry step. | | |

**TABLE II**

| DESIGN SPECIFICATIONS, COMMERCIAL FACILITY WATER PRETREATMENT UNIT (SERVES 1 PHOTOSYNTHETRON). | | |
|---|---|---|
| I. | STATIC | Dimensions (MKS) |
| | Length | 350 m |
| | Width | 80 m |
| | Depth | 1.5 m |
| | Plan area | 14,000 m² |
| | Volume | 40,600 m³ |
| | Number of compartments | 2 |
| | Volume of one compartment | 20,300 m³ |

| II. | DYNAMIC | |
|---|---|---|
| | Filling rate | 14.10 m³/min |
| | Filling time | 24 h |
| | Emptying rate | 104.8 m³/min |
| | Emptying time | 3.23 h |
| | Heating rate (average) | 5.4 C/day |
| | Heating rate (peak power) | 10.8 C/day |

**TABLE III**

| GROWTH MODULE - DESIGN SPECIFICATION | |
|---|---|
| STATIC | Dimensions (MKS) |
| Length (overall) | 30 m |
| Length (growth chamber) | 27.5 m |
| Width | 10 m |
| Plan area (slab) | 300 m² |
| Plan area (growth chamber) | 275 m² |
| Growth tube diameter | 20.3 cm |
| Number of Tubes | 4o |
| Number of 180 U-joints | 39 |
| Cumulative tube length | 1,100 m |
| Cumulative tube width | 8.12 m |
| Tube plan area | 223 m² |
| Tube volume | 35.6 m³ |
| Reservoir volume | 2.0 m³ |
| Total fluid volume | 37.6 m³ |
| Concrete volume (311 slab) | 22.8 m³ |
| Air Volume | 1,178 m³ |
| Thermal Mass of Module: | |
| Water | 87,582 BTU/F |
| Concrete | 24,210 BTU/F |
| Air | 773 BTU/F |

| DYNAMIC | |
|---|---|
| Evaporation | 0 |
| Flow rate (linear) | 10 cm/s |
| Flow rate (volumetric) | 194 L/min |
| Reynolds number | 20,000 |
| Recycle time | 193.6 min |
| Harvest volume | 18.8 m³ |
| Harvest time | 193.6 min |
| Harvest flow rate | 97 L/min |
| Cycles per growth period | 3.72 |
| Harvests per day | 1 |

**TABLE IV**

| SEDIMENT TANK AND HARVESTING SYSTEM DESIGN SPECIFICATION | |
|---|---|
| STATIC | Dimensions (MKS) |
| Length (overall) | 180 m |
| Width | 4.8 m |
| Depth | 4.2 m |
| Angle of incline (degrees) | 60 |
| Plan area | 864 m² |
| Volume (total) | 1,814 m³ |
| Number of growth modules serviced | 90 |
| Area of concrete slab | 1,763 m² |
| Concrete volume (311 slab) | 134.3 m³ |
| Inflow pipe diameter | 15-42 cm |
| Number of electroflocculators | 6 |
| Number of output floc pumps | 6 |
| Output floc pipe diameter | 3.8 cm |
| Number of supernatant outputs | 6 |
| Supernatant output pipe diam. | 15.24 cm |
| Module cover area | 1,080 m² |

| DYNAMIC | |
|---|---|
| on per-pipe basis: Input flow rate (linear) | 134 cm/s |
| Input flow rate (volumetric) | 1,461 L/min |
| Input Reynolds number | 205,000 |
| Floc output flow rate (linear) | 46.2 cm/s |
| Floc output flow rate (vol.) | 31.6 L/min |
| Floc output Reynolds number | 17,600 |
| Supernatant flow rate (linear) | 130-288 cm/s |
| Supernatant flow rate (vol. | 1420-3145 L/m |
| Supernatant output Reynolds Number | 200,000-400,000 |
| at Peak Power on per-tank basis: | 2.8-5.6 12C/h |
| Input flow rate (linear) | 134 cm/s |
| Input flow rate (volumetric) | 8,766 L/min |
| Input Reynolds number | 205,00 |
| Floc output flow rate (linear) | 277 cm/s |
| Floc output flow rate (vol.) | 190 L/min |
| Floc output Reynolds number | 105,000 |
| Supernatant flow rate (linear) | 7.8-17.3 m/s |
| Supernatant flow rate (vol.) | 8.5-18.9 m³/m |
| Supernatant output Reynolds Number | 1,200,000-2,600,000 |
| sedimentation rate | 0.5-2.0 m/h |
| Fill time | 3.23 h |
| Sedimentation time | 2.00-8.00 h |
| Floc output time (25X conc. | 5.95 h |
| Floc output time (50X conc. | 2.97 h |
| Supernatant output time (@ 4000 gpm) | 1.79 h |
| Minimum cycle time | 600 min |
| Maximum cycle time | 1,138 min |
| Centrifuge operating capacity | 1,895 L/min |
| Harvests per day | 1 |
| Evaporation | 0 |

## Claims

1. A method for culturing photosynthetic microbes and cells of photosynthetic macrophytes in a closed, continuous, circulating system, comprising:
a) pretreating source water (22) to be used as culture medium;
b) circulating said pretreated culture water (22) through a closed, continuous, conduit which is substantially horizontally disposed and the walls of which are transparent to sunlight within a protective growth module which is also transparent to sunlight;
c) inoculating said circulating culture water with cells of a photosynthetic species;
d) producing a substantially continuous flow regime having a Reynolds Number of at least 2000 in said circulating culture water (22);
e) monitoring turbulence and one or more physical, chemical or biological properties of said circulating culture water (22), containing photosynthetic cells within said closed conduit;
f) automatically comparing the value of said observed physical, chemical or biological properties of said circulating culture water to a predetermined optimum value for said property;
g) automatically adjusting said physical, chemical or biological property of said circulating culture water, when said observed value deviates from said optimum value;
h) isolating a concentrated portion of photosynthetic cells grown in said conduit from said circulating culture water without affecting total volume; and
i) separating and recovering said photosynthetic cells from said circulating culture water.

2. The method of Claim 1, wherein said pretreatment of said source water in step (a) includes solar preheating.

3. The method of Claim 1, wherein said pretreatment of said source water in step (a) further includes initial addition of nutrients (55) prior to circulation.

4. The method of Claim 1, wherein said pretreatment of said source water in step (a) includes sterilization (16).

5. The method of Claim 1, wherein said inoculation of said closed conduit of one growth module in step (c) is caused by removal of a portion of said circulating culture water in said closed conduit of another growth module, and its introduction into said closed conduit of said first growth module, so as to effect immediate full operating capacity of said first growth module.

6. The method of Claim 1, wherein a second property which is monitored, compared and automatically adjusted in steps (e), (f) and (g) is the pH of said circulating culture water.

7. The method of Claim 1, wherein a second property which is monitored, compared and automatically adjusted in steps (e), (f) and (g) is the CO₂ concentration of said circulating culture water.

8. The method of Claim 1, wherein a second property which is monitored, compared and automatically adjusted in steps (e), (f) and (g) is the temperature of said circulating culture water.

9. The method of Claim 1, wherein a second property which is monitored, compared and automatically adjusted in steps (e), (f) and (g) is the cell concentration in said circulating culture water.

10. The method of Claim 1, wherein a second property which is monitored in step (e) is productivity of said photosynthetic cells contained in said circulating culture water.

11. The method of Claim 1, wherein said isolation and separation of said photosynthetic cells in steps (h) and (i) is accomplished by removing a portion of said circulating culture water from said closed conduit, flocculating cellular particulates contained therein and separating said flocculated cellular particulates from supernatant water by sedimentation.

12. The method of Claim 11, wherein said separated flocculated cellular particulates are centrifuged to provide further separation from supernatant water.

13. The method of Claim 12, wherein said centrifuged flocculated cellular particulates are subjected to solar drying to produce a dried biomass.

14. The method of Claim 1, wherein said isolation and recovery of photosynthetic cells in step (h) is accomplished by removing a portion of said circulating culture water and providing it directly, without separating or concentrating said photosynthetic cells, to living aquatic organisms which require said photosynthetic cells as food.

15. Apparatus for large-scale culturing of photosynthetic cells of a desired species, including microbes and cells of photosynthetic macrophytes, in a closed, continuous, circulating system, comprising:
a water pumping and filtration system (1) supplying pretreated culture water for growing a culture of photosynthetic cells;
at least one growth module (18) containing a continuous, closed culture conduit substantially horizontally disposed for circulating culture water (22), the walls of said conduit being transparent to light, the conduit being disposed within a protective growth module which is transparent to sunlight;
means (63) for circulating said culture water through said closed culture conduit;
means for inoculating said circulating culture water (22) with a cell mass of said photosynthetic cell species to form a concentration thereof within said culture water;
means for monitoring turbulence (81,83) and one other physical, chemical or biological property (71, 73, 75, 77) of said culture water circulating within said closed culture conduit, and comparing the observed values of said monitored properties of said circulating culture water to a predetermined optimum value for each said property;
means (63) for producing a substantially continuous flow regime having a Reynolds Number of at least 2000 in said circulating culture water;
means responsive to said comparison of said observed and optimum turbulence values for changing the Reynolds Number of said turbulent flow regime;
output means (67) for isolating a portion of said circulating culture water containing said cell mass from said conduit without affecting the level of culture-containing circulating water;
input means (65) for introducing and maintaining an appropriate level of said culture water into said conduit; and
means for separating said photosynthetic cells from said circulating culture water isolated by said output means.

16. The apparatus of Claim 15, wherein said system of supplying pretreated source water comprises an aquifer (22), a reservoir (8), a filter (6), a mixing tank (51) and optional pump means (4, 12) situated in a conduit connecting these together in series and to said closed culture conduit.

17. The apparatus of Claim 15, wherein said photosynthetic cell culture growth module (18) is enclosed within a transparent housing (23).

18. The apparatus of Claim 17, wherein said transparent housing (23) is further enclosed by another transparent housing which comprises at least one layer of polyethylene or comparable plastic material supported by a rigid frame structure.

19. The apparatus of Claim 15, wherein said closed conduit for circulating water comprises a series of tubular members (33) disposed substantially horizontally side by side parallel to one another and joined to adjacent members by 180° U-shaped tubular returns (49).

20. The apparatus of claim 19, wherein said tubular members (33) are made from a material selected from the group consisting of polyethylene, polypropylene, polyacrylates, polamides, polycarbonates, water-insoluble cellulose esters, polyester films and glass.

21. The apparatus of Claim 19, wherein the means for joining said parallel tubular members (33) to said 180° U-shaped tubular returns (49) is thermal fusion, achieved by the application of uniform heat of a temperature appropriate to melt the material being used.

22. The apparatus of Claim 15, wherein said means for circulating water through said closed conduit is partly accomplished by a minor slope of said closed conduit, such that gravitational forces overcome dynamic head pressure generated within said conduit system.

23. The apparatus of Claim 15, wherein said means for inoculating said circulating water with cells of said desired species so as to rapidly achieve full operating capacity comprises a tubular member joining independent closed conduits.

24. The apparatus of Claim 23, wherein said means for inoculating is by introducing from an independent photosynthetic cell culture means a portion of its circulating water with cells of said desired species, through said tubular joining member, to said photosynthetic cell culture means being inoculated.

25. The apparatus of Claim 15, wherein said means (71, 73, 75, 77) for monitoring two or more physical, chemical or biological properties of said circulating water comprises one or more probes inserted into said closed conduit for circulating water.

26. The apparatus of Claim 25, wherein the monitored physical, chemical or biological property other than degree of turbulence is said concentration of photosynthetic cells in said circulating water and said means for monitoring is an optical sensor (71) capable of measuring light transmission or light absorption of said circulating water, or light emission of said photosynthetic cells.

27. The apparatus of Claim 25, wherein the means for comparing said observed physical, chemical or biological properties to predetermined optimum values for said properties is a microprocessor (79) and appropriate software.

28. The apparatus of Claim 27, wherein said monitored physical, chemical or biological property other than degree of turbulence is pH, CO₂ or temperature.

29. The apparatus of Claim 27, wherein said monitored physical, chemical or biological property is pH and said means for automatically adjusting pH to a lower value is a pressurized CO₂ tank having a valve which is activated by a signal from said microprocessor (79) to introduce CO₂ into said circulating water (22).

30. The apparatus of Claim 27, wherein said monitored physical, chemical or biological property is CO₂ and said means for automatically adjusting CO₂ content is a pressurized CO₂ tank having a valve which is activated by a signal from said microprocessor to introduce CO₂ into said circulating water.

31. The apparatus of Claim 27, wherein said monitored physical, chemical or biological property is temperature and said means for automatically adjusting said temperature is an exhaust fan (31) positioned above said closed conduit for circulating water which is activated by a signal from said microprocessor.

32. The apparatus of Claim 27, wherein said monitored physical, chemical or biological property is temperature and said means for automatically adjusting temperature is a sprinkler system, suspended above said photosynthetic cell culture means, having a valve which is activated by a signal from said microprocessor to release droplets of water onto said closed conduit for circulating water.

33. The apparatus of Claim 27, wherein said monitored physical, chemical or biological property is temperature and said means for automatically adjusting temperature is fresh input water, of a lower temperature than that contained in the photosynthetic cell culture means, released into said closed conduit for circulating water through a valve which is activated by said microprocessor.

34. The apparatus of Claim 27, wherein said monitored physical, chemical or biological property is productivity of photosynthetic cells, and said means for automatically adjusting productivity is by altering one or more of pH, CO₂ temperature, nutrient content and said input of fresh water into said circulating water.

35. The apparatus of Claim 15, further comprising means for purifying and recycling to said source of water said isolated culture water separated from said photosynthetic cells.

## Patentansprüche

1. Verfahren zur Kultivierung photosynthetischer Mikroben und Zellen photosynthetischer Makrophyten in einem geschlossenen durchgehenden Zirkulationssystem, bei dem
a) als Kulturmedium zu verwendendes Ausgangswasser (22) vorbehandelt wird;
b) das vorbehandelte Kulturwasser (22) durch eine geschlossene durchgehende Leitung, die im wesentlichen horizontal angeordnet ist und deren Wände für Sonnenlicht durchlässig sind, innerhalb eines schützenden Wachstumsmoduls zirkuliert wird, das ebenfalls für Sonnenlicht durchlässig ist;
c) das zirkulierende Kulturwasser mit Zellen photosynthetischer Spezien geimpft wird;
d) in dem zirkulierenden Kulturwasser (22) ein Bereich im wesentlichen durchgehender Strömung mit einer Reynoldszahl von wenigsten 2.000 erzeugt wird;
e) eine Turbulenz und eine oder mehrere physikalische, chemische oder biologische Eigenschaften des zirkulierenden Kulturwassers (22) innerhalb der geschlossenen Leitung überwacht wird, das photosynthetische Zellen enthält;
f) der Wert der beobachteten physikalischen, chemischen oder biologischen Eigenschaften des zirkulierenden Kulturwassers mit einem vorher bestimmten optimalen Wert für diese Eigenschaften automatisch verglichen wird;
g) die physikalische, chemische oder biologische Eigenschaft des zirkulierenden Kulturwassers automatisch eingestellt wird, wenn der beobachtete Wert von dem optimalen Wert abweicht;
h) ein konzentrierter Teil von photosynthetischen Zellen, die in der Leitung aus dem zirkulierenden Kulturwasser gewachsen sind, ohne Auswirkung auf das Gesamtvolumen
isoliert wird; und i) die photosynthetischen Zellen aus dem zirkulierenden Kulturwasser abgetrennt und rückgewonnen werden.

2. Verfahren nach Anspruch 1, bei dem die Vorbehandlung des Ausgangswassers in Schritt (a) eine solare Vorwärmung umfaßt.

3. Verfahren nach Anspruch 1, bei dem die Vorbehandlung des Ausgangswassers in Schritt (a), außerdem die anfängliche Zugebung von Nährstoffen (55) vor der Zirkulierung umfaßt.

4. Verfahren nach Anspruch 1, bei dem die Vorbehandlung des Ausgangswassers in Schritt (a) eine Sterilisierung (16) umfaßt.

5. Verfahren nach Anspruch 1, bei dem die Impfung der geschlossenen Leitung eines Wachstumsmoduls in Schritt (c) durch Entfernung eines Teils des zirkulierenden Kulturwassers in der geschlossenen Leitung eines anderen Wachstumsmoduls und seine Einführung in die geschlossene Leitung des ersten Wachstumsmoduls verursacht wird, um eine sofortige volle Betriebskapazität des ersten Wachstumsmoduls zu erreichen.

6. Verfahren nach Anspruch 1, bei dem eine zweite Eigenschaft, die in den Schritten (e), (f) und (g) überwacht, verglichen und automatisch eingestellt wird, der pH-Wert des zirkulierenden Kulturwassers ist.

7. Verfahren nach Anspruch 1, bei dem eine zweite Eigenschaft, die in den Schritten (e), (f) und (g) überwacht, verglichen und automatisch eingestellt wird, die CO₂-Konzentration des zirkulierenden Kulturwassers ist.

8. Verfahren nach Anspruch 1, bei dem eine zweite Eigenschaft, die in den Schritten (e), (f) und (g) überwacht, verglichen und automatisch eingestellt wird, die Temperatur des zirkulierenden Kulturwassers ist.

9. Verfahren nach Anspruch 1, bei dem eine zweite Eigenschaft, die in den Schritten (e), (f) und (g) überwacht, verglichen und automatisch eingestellt wird, die Zellkonzentration in dem zirkulierenden Kulturwassers ist.

10. Verfahren nach Anspruch 1, bei dem eine zweite Eigenschaft, die in Schritt (e) überwacht wird, die Produktivität der photosynthetischen Zellen ist, die in dem zirkulierenden Kulturwasser enthalten sind.

11. Verfahren nach Anspruch 1, bei dem die Isolierung und Abtrennung der photosynthetischen Zellen in den Schritten (h) und (i) durch Entfernung eines Teils des zirkulierenden Kulturwassers aus der geschlossenen Leitung, durch Ausflockung zellularer Partikel, die darin enthalten sind, und durch Abtrennung der ausgeflockten zellularen Partikel aus überschüssigem Wasser durch Sedimentation durchgeführt wird.

12. Verfahren nach Anspruch 11, bei dem die abgetrennten ausgeflockten zellularen Partikel zentrifugiert werden, um eine weitere Abtrennung von überschüssigem Wasser zu schaffen.

13. Verfahren nach Anspruch 12, bei dem die zentrifugierten, ausgeflockten zellularen Partikel einer Solartrocknung unterworfen werden, um eine getrocknete Biomasse zu erzeugen.

14. Verfahren nach Anspruch 1, bei dem die Isolierung und Rückgewinnung photosynthetischer Zellen in Schritt (h) dadurch durchgeführt wird, daß ein Teil des zirkulierenden Kulturwassers entfernt wird und es direkt, ohne Abtrennung oder Konzentrierung der photosynthetischen Zellen, mit lebenden Wasserorganismen versehen wird, die die photosynthetischen Zellen als Futter verwenden.

15. Vorrichtung zur Kultivierung von photosynthetischen Zellen einer gewünschten Spezies, einschließlich Mikroben und Zellen photosynthetischer Makrophyten, in großem Maßstab in einem geschlossenen durchgehenden Zirkulationssystem, mit
- einem Wasserpump- und Filtriersystem (1) zur Zuführung von vorbehandeltem Kulturwasser zur Züchtung einer Kultur photosynthetischer Zellen;
- wenigstens einem Wachstumsmodul (18), das eine durchgehende geschlossene Kulturleitung enthält, die für eine Zirkulierung von Kulturwasser (22) im wesentlichen horizontal angeordnet ist, wobei die Wände der Leitung für Licht durchlässig sind, und die Leitung innerhalb eines schützenden Wachstumsmoduls angeordnet ist, das für Sonnenlicht durchlässig ist;
- Mitteln (63) für eine Zirkulierung des Kulturwassers durch die geschlossene Kulturleitung;
- Mitteln zur Impfung des zirkulierenden Kulturwassers (22) mit einer Zellmasse aus den photosynthetischen Zellspezien zur Bildung einer Konzentration hiervon innerhalb des Kulturwassers;
- Mitteln zur Beobachtung einer Turbulenz (81,83) und einer physischen, chemischen oder biologischen Eigenschaft (71,73,75,77) des innerhalb der geschlossenen Kulturleitung zirkulierenden Kulturwassers und zum Vergleich der beobachteten Werte der beobachteten Eigenschaften des zirkulierenden Kulturwassers mit einem vorher bestimmten optimalen Wert für jede Eigenschaft;
- Mitteln (63) zur Erzeugung eines Bereichs mit einer im wesentlichen durchgehenden Strömung mit einer Reynoldszahl von wenigsten 2.000 in dem zirkulierenden Kulturwasser;
- Mitteln, die zur Veränderung der Reynoldszahl des Bereiches mit turbulenter Strömung auf den Vergleich der beobachteten und optimalen Turbulenzwerte reagieren;
- Ausgabemitteln (67) für eine Isolierung eines Teils des die Zellmasse enthaltenden zirkulierenden Kulturwassers aus der Leitung ohne Auswirkung auf den Gehalt eine Kultur enthaltenden zirkulierenden Wassers;
- Eingabemitteln (65) für eine Einführung und Aufrechterhaltung eines geeigneten Gehalts des Kulturwassers in der Leitung; und
- Mitteln zur Abtrennung der photosynthetischen Zellen aus dem zirkulierenden Kulturwasser, das durch die Abgabemittel isoliert wurde.

16. Vorrichtung nach Anspruch 15, bei der das System zur Zufuhr von vorbehandeltem Ausgangswasser einen Aquifer (22), ein Reservoir (8), einen Filter (6), einen Mischtank (51) und eine optionale Pumpeinrichtung (4, 12 ) umfaßt, die in einer Leitung angeordnet ist, die diese miteinander in Reihe und mit der geschlossenen Kulturleitung verbindet.

17. Vorrichtung nach Anspruch 15, bei der das Wachstumsmodul (18) für die photosynthetische Zellkultur von einem lichtdurchlässigen Gehäuse (23) umschlossen ist.

18. Vorrichtung nach Anspruch 17, bei der das lichtdurchlässige Gehäuse (23) weiterhin durch ein anderes lichtdurchlässiges Gehäuse umschlossen ist, das wenigsten eine Schicht aus Polyethylen oder vergleichbarem Kunststoffmaterial umfaßt, die von einem steifen Rahmenaufbau getragen ist.

19. Vorrichtung nach Anspruch 15, bei der die geschlossene Leitung für die Zirkulierung von Wasser eine Reihe von Rohrelementen (33) umfaßt, die im wesentlichen horizontal und seitlich parallel zueinander angeordnet sind und mit angrenzenden Elementen durch um 180° gebogenen U-förmige Rohrrücklaufe (49) verbunden sind.

20. Vorrichtung nach Anspruch 19, bei der die Rohrelemente (33) aus einem Material hergestellt sind, das aus einer Gruppe ausgewählt ist, die aus Polyethylen, Polypropylen, Polyacrylaten, Polyamiden, Polycarbonaten, wasserlöslichen Zelluloseestern, Polyesterfilmen und Glas besteht.

21. Vorrichtung nach Anspruch 19, bei der das Mittel zur Verbindung der parallelen Rohrelemente (33) mit den um 180° gebogenen U-förmigen Rohrrückläufen (49) eine thermische Fusion ist, die durch Aufbringung einer gleichmäßigen Wärme mit einer Temperatur erreicht wird, die dazu geeignet ist, das verwendete Material zu schmelzen.

22. Vorrichtung nach Anspruch 15, bei der das Mittel zur Zirkulierung von Wasser durch die geschlossene Leitung teilweise durch eine geringe Neigung der geschlossenen Leitung verwirklicht wird, so daß die Gravitationskräfte den Druck des Druckgefälles überwinden können, das innerhalb des Leitungssystems erzeugt wird.

23. Vorrichtung nach Anspruch 15, bei der das Mittel zur Impfung des zirkulierendes Wassers mit Zellen der gewünschten Spezies zur raschen Erreichung einer vollen Betriebskapazität ein Rohrelement umfaßt, das unabhängige geschlossene Leitungen miteinander verbindet.

24. Vorrichtung nach Anspruch 23, bei der als Mittel zur Impfung ein Teil zirkulierenden Wassers mit Zellen der gewünschten Spezies aus einer unabhängigen photosynthetischen zellkultureinrichtung über das rohrförmige Verbindungselement in die zu impfende photosynthetische Zellkultureinrichtung eingeführt wird.

25. Vorrichtung nach Anspruch 15, bei der das Mittel (71,73, 75,77) zur Überwachung zweier oder mehrerer physikalischer, chemischer oder biologischer Eigenschaften des zirkulierenden Wassers eine oder mehrere Proben umfaßt, die in die geschlossene Leitung für die Zirkulierung von Wasser eingesetzt sind.

26. Vorrichtung nach Anspruch 25, bei der die nicht den Turbulenzgrad darstellende überwachte physikalische, chemische oder biologische Eigenschaft die Konzentration der photosynthetischen Zellen in dem zirkulierenden Wasser ist, und das Mittel zur Überwachung ein optischer Sensor (71) ist, der eine Lichtübertragung oder Lichtabsorption des zirkulierenden Wassers oder eine Lichtemission der photosynthetischen Zellen messen kann.

27. Vorrichtung nach Anspruch 25, bei der das Mittel zum Vergleich der beobachteten physikalischen, chemischen oder biologischen Eigenschaften mit vorher bestimmten optimalen Werten für die Eigenschaften ein Mikroprozessor (79) und eine geeignete Software ist.

28. Vorrichtung nach Anspruch 27, bei der die überwachte physikalische, chemische oder biologische Eigenschaft, die nicht den Turbulenzgrad darstellt, ein Ph-Wert, ein CO₂-Wert oder eine Temperatur ist.

29. Vorrichtung nach Anspruch 27, bei der die überwachte physikalische, chemische oder biologische Eigenschaft ein Ph-Wert ist und das Mittel zur automatischen Einstellung des Ph-Werts auf einen unteren Wert ein unter Druck gesetzter CO₂-Behalter mit einem Ventil ist, das durch ein Signal von dem Mikroprozessor (79) aktiviert wird, um CO₂ in das zirkulierende Wasser (22) einzuführen.

30. Vorrichtung nach Anspruch 27, bei der die überwachte physikalische, chemische oder biologische Eigenschaft ein CO₂-Wert ist und das Mittel zur automatischen Einstellung des CO₂-Gehalts ein unter Druck gesetzter CO₂-Tank mit einem Ventil ist, das durch ein Signal von dem Mikroprozessor aktiviert wird, um CO₂ in das zirkulierende Wasser einzuführen.

31. Vorrichtung nach Anspruch 27, bei der die überwachte physikalische, chemische oder biologische Eigenschaft eine Temperatur ist und das Mittel zur automatischen Einstellung der Temperatur ein Abluftventilator ist, der oberhalb der geschlossenen Leitung für zirkulierendes Wasser angeordnet ist und durch ein Signal von dem Mikroprozessor aktiviert wird.

32. Vorrichtung nach Anspruch 27, bei der die überwachte physikalische, chemische oder biologische Eigenschaft eine Temperatur ist und das Mittel zur automatischen Einstellung der Temperatur ein Sprinklersystem ist, das oberhalb der photosynthetischen Zellkultureinrichtung aufgehängt ist und ein Ventil aufweist, das durch ein Signal von dem Mikroprozessor aktiviert wird, um Wassertropfen auf die geschlossene Leitung für zirkulierendes Wasser abzugeben.

33. Vorrichtung nach Anspruch 27, bei der die überwachte physikalische, chemische oder biologische Eigenschaft eine Temperatur ist und das Mittel zur automatischen Einstellung der Temperatur frisches Zugabewasser ist, das eine geringere Temperatur als das in der photosynthetischen Zellkultureinrichtung enthaltene hat und in die geschlossenen Leitung für zirkulierendes Wasser über ein Ventil abgegeben wird, das durch den Mikroprozessor aktiviert wird.

34. Vorrichtung nach Anspruch 27, bei der die überwachte physikalische, chemische oder biologische Eigenschaft eine Produktivität der photosynthetischen Zellen ist und das Mittel zur automatischen Einstellung der Produktivität die Veränderung eines oder mehrerer Werte von Ph, CO₂, Temperatur, Nährstoffgehalt und Zugabe von frischem Wasser in das zirkulierende Wasser ist.

35. Vorrichtung nach Anspruch 15, die außerdem Mittel zur Reinigung des von den photosynthetischen Zellen getrennten isolierten Kulturwassers und zu dessen Rückführung zur Wasserquelle umfaßt.

## Revendications

1. Procédé de culture de microbes photosynthétiques et de cellules à macrophytes photosynthétiques dans un système fermé, continu et circulant, comprenant :
a) le prétraitement de la source d'eau (22) utilisée en tant que milieu de culture ;
b)la circulation de l'eau de culture prétraitée (22) à travers un conduit fermé et continu, qui est disposé sensiblement de façon horizontale et dont les parois sont transparentes à la lumière du soleil, à l'intérieur d'un module de croissance protecteur (qui est) également transparent à la lumière du soleil ;
c) l'inoculation de cellules d'espèces photosynthétiques à l'eau de culture circulante ;
d) la production d'un régime d'écoulement sensiblement continu dont le Nombre de Reynolds est au moins 2000, dans l'eau de culture circulante (22) ;
e) la mesure de la turbulence et d'une ou de plusieurs caractéristiques physiques, chimiques ou biologiques de l'eau de culture circulante (22) contenant des cellules photosynthétiques à l'intérieur du conduit fermé ;
f) la comparaison automatique de la valeur des caractéristiques physiques, chimiques ou biologiques observées de l'eau de culture circulante avec une valeur optimale de la caractéristique ;
g) l'ajustement automatique de la caractéristique physique, chimique ou biologique de l'eau de culture circulante lorsque la valeur observée s'écarte de la valeur optimale ;
h) l'isolement à partir de l'eau de culture circulante d'une partie concentrée de cellules photosynthétiques croissant dans le conduit, sans affecter le volume total ; et
i) la séparation et la récupération des cellules photosynthétiques de l'eau de culture circulante.

2. Procédé selon la revendication 1,
caractérisé en ce que
le prétraitement de la source d'eau dans l'étape (a) comprend un préchauffage solaire.

3. Procédé selon la revendication 1,
caractérisé en ce que
le prétraitement de la source d'eau dans l'étape (a) comprend en plus l'ajout initial de nutriments (55) avant la mise en circulation.

4. Procédé selon la revendication 1,
caractérisé en ce que
le prétraitement de la source d'eau dans l'étape (a) comprend une stérilisation (16).

5. Procédé selon la revendication 1,
caractérisé en ce que
l'inoculation du conduit fermé d'un des modules de croissance dans l'étape (c) est due au déplacement d'une partie de l'eau de culture circulante dans le conduit fermé d'un autre module de croissance, et à son introduction dans le conduit fermé du premier module de croissance, afin de réaliser une immédiate complète capacité de fonctionnement dans le premier module de croissance.

6. Procédé selon la revendication 1,
caractérisé en ce qu'
une seconde caractéristique mesurée, comparée et automatiquement ajustée dans les étapes (e), (f) et (g) est le pH de l'eau de culture circulante.

7. Procédé selon la revendication 1,
caractérisé en ce qu'
une seconde caractéristique mesurée, comparée et automatiquement ajustée dans les étapes (e), (f) et (g) est la concentration de CO₂ de l'eau de culture circulante.

8. Procédé selon la revendication 1,
caractérisé en ce qu'
une seconde caractéristique mesurée, comparée et automatiquement ajustée dans les étapes (e), (f) et (g) est la température de l'eau de culture circulante.

9. Procédé selon la revendication 1,
caractérisé en ce qu'
une seconde caractéristique mesurée, comparée et automatiquement ajustée dans les étapes (e), (f) et (g) est la concentration cellulaire de l'eau de culture circulante.

10. Procédé selon la revendication 1,
caractérisé en ce qu'
une seconde caractéristique mesurée dans l'étape (e) est le rendement des cellules photosynthétiques contenues dans l'eau de culture circulante.

11. Procédé selon la revendication 1,
caractérisée en ce que
l'isolement et séparation des cellules photosynthétiques dans les étapes (h) et (i) est effectué par déplacement d'une partie de l'eau de culture circulante du conduit fermé, par floculation des particules cellulaires contenues à l'intérieur et par séparation des particules cellulaires floculées par sédimentation de l'eau du surnageant.

12. Procédé selon la revendication 11,
caractérisé en ce que
les particules cellulaires floculées séparées sont centrifugées pour obtenir une séparation ultérieur à partir de l'eau du surnageant.

13. Procédé selon la revendication 12,
caractérisé en ce que les particules cellulaires floculées centrifugées sont soumises à une déshydratation solaire afin d'obtenir une biomasse sèche.

14. Procédé selon la revendication 1,
caractérisé en ce que
l'isolement et récupération des cellules photosynthétiques dans l'étape (h) est effectué par déplacement d'une partie de l'eau de culture circulante et son utilisation directe, sans effectuer de séparation ou de concentration des cellules photosynthétiques, pour des organismes aquatiques vivants qui ont besoin pour nourriture des cellules photosynthétiques.

15. Appareil de culture à grande échelle de cellules photosynthétiques d'espèces désirées, incluant les microbes et les cellules à macrophytes photosynthétiques, dans un système fermé, continu et circulant, comprenant :
• une pompe à eau et un système de filtration (1) alimentant l'eau de culture prétraitée pour la croissance d'une culture de cellules photosynthétiques ;
• au moins un module de croissance (18) contenant un conduit de culture continu et fermé, disposé sensiblement de façon horizontale par rapport à l'eau de culture circulante (22), les parois du conduit étant transparentes à la lumière du soleil, le conduit étant disposé à l'intérieur d'un module de croissance protecteur qui est transparent à la lumière du soleil ;
• le moyen (63) pour la circulation de l'eau de culture à travers le conduit de culture fermé ;
• le moyen pour l'inoculation d'amas cellulaire des espèces cellulaires photosynthétiques à l'eau de culture circulante (22) pour obtenir une concentration de celles-ci dans l'eau de culture ;
• les moyens (81,83) pour mesurer la turbulence et une autre caractéristique physique, chimique ou biologique (71,73,75,77) de l'eau de culture circulante à l'intérieur du conduit de culture fermé, et comparer les valeurs observées des caractéristiques mesurées de l'eau de culture circulante à une valeur optimale prédéterminée pour chaque caractéristique ;
• le moyen (63) pour produire un régime à flux sensiblement continu, dont le Nombre de Reynolds est au moins 2000, dans l'eau de culture circulante;
• le moyen sensible à la comparaison de la valeur de la turbulence observée et optimale qui permet de changer le Nombre de Reynolds du régime d'écoulement turbulent ;
• le moyen de sortie (67) pour isoler du conduit une partie de l'eau de culture circulante contenant le amas cellulaire sans altérer le niveau de l'eau circulante contenant la culture ;
• le moyen d'entrée (65) pour introduire et maintenir un niveau approprié de l'eau de culture dans le conduit ; et
• le moyen pour séparer les cellules photosynthétiques, isolées par le moyen de sortie, de l'eau de culture circulante.

16. Appareil selon la revendication 15,
caractérisé en ce que
le système pour fournir l'eau de source prétraitée comprend un aquifère (22), un réservoir (8) , un filtre (6) , une cuve-mélangeur (51) et des moyens de pompage optionnels (4,12) situés dans un conduit les reliant ensemble en série et au conduit de culture fermé.

17. Appareil selon la revendication 15,
caractérisé en ce que
le module de croissance de la culture cellulaire photosynthétique (18) est enfermé à l'intérieur d'un habitacle transparent (23).

18. Appareil selon la revendication 17,
caractérisé en ce que
l'habitacle transparent (23) est enfermé encore dans un autre habitacle transparent qui comprend au moins une couche de polyéthylène ou d'un matériau plastique comparable soutenue par une ossature rigide.

19. Appareil selon la revendication 15,
caractérisé en ce que
le conduit fermé pour la circulation de l'eau comprend une série de membres tubulaires (33) disposés sensiblement de façon horizontale, côte à côte, parallèles l'un à l'autre et réunis aux membres adjacents par une extrémité tubulaire en forme de U de 180°.

20. Appareil selon la revendication 19,
caractérisé en ce que
les membres tubulaires (33) sont fabriqués à partir d'un matériau choisi parmi le groupe constitué de polyéthylène, de polypropylène, de polyacrylates, de polyamides, de polycarbonates, d'esters de cellulose insolubles dans l'eau, de membranes de polyester et de verre.

21. Appareil selon la revendication 19,
caractérisé en ce que
le moyen pour réunir les membres tubulaires parallèles (33) aux extrémités tubulaires en forme de U de 180° (49) est à fusion thermique, ce moyen est réalisé par l'application d'une chaleur uniforme d'une température appropriée pour fondre le matériau utilisé.

22. Appareil selon la revendication 15,
caractérisé en ce que
le moyen pour la mise en circulation de l'eau à travers le conduit fermé est effectué en partie par une inclinaison mineure du conduit fermé, afin que les forces gravitationnelles surmontent la pression dynamique principale générée à l'intérieur du système de conduits.

23. Appareil selon la revendication 15,
caractérisé en ce que
le moyen d'inoculation des cellules des espèces désirées à l'eau circulante, afin d'atteindre rapidement une complète capacité de fonctionnement, comprend un élément tubulaire reliant les conduits fermés autonomes.

24. Appareil selon la revendication 23,
caractérisé en ce que
le moyen pour inoculer s'effectue par l'introduction, à partir de cultures de cellules photosynthétiques indépendante au moyen d'une partie de l'eau circulante avec des cellules d'espèces désirées de liaison, ayant été inoculées au moyen de cultures de cellules photosynthétiques à travers l'élément tubulaire.

25. Appareil selon la revendication 15,
caractérisé en ce que
les moyens (71, 73, 75, 77) pour mesurer deux ou plusieurs caractéristiques physiques, chimiques ou biologiques de l'eau circulante comprennent un ou plusieurs capteurs insérés à l'intérieur du conduit de circulation de l'eau.

26. Appareil selon la revendication 25,
caractérisé en ce que
la caractéristique physique, chimique ou biologique mesurée est la concentration des cellules photosynthétiques dans l'eau circulante, et le moyen pour mesurer est un capteur optique (71) capable de mesurer la transmission de la lumière ou l'absorption de la lumière de l'eau circulante, ou l'émission lumineuse des cellules photosynthétiques.

27. Appareil selon la revendication 25,
caractérisé en ce que
le moyen pour comparer les caractéristiques physiques, chimiques ou biologiques aux valeurs optimales prédéterminées pour les caractéristiques est un microprocesseur (79) et un logiciel approprié.

28. Appareil selon la revendication 27,
caractérisé en ce que
la caractéristique physique, chimique ou biologique mesurée, autre que le degré de turbulence, est le pH, le CO₂ ou la température.

29. Appareil selon la revendication 27,
caractérisé en ce que
la caractéristique physique, chimique ou biologique mesurée est le pH, et le moyen pour ajuster automatiquement le pH à une valeur inférieur est un réservoir à CO₂ sous pression possédant une soupape activée par un signal provenant du microprocesseur (79) afin d'introduire du CO₂ dans l'eau circulante (22).

30. Appareil selon la revendication 27,
caractérisé en ce que
la caractéristique physique, chimique ou biologique mesurée est le CO₂, et le moyen pour régler automatiquement le contenu en CO₂ est un réservoir à CO₂ sous pression possédant une soupape qui est activée par un signal provenant du microprocesseur (79) afin d'introduire du CO₂ dans l'eau circulante.

31. Appareil selon la revendication 27,
caractérisé en ce que
la caractéristique physique, chimique ou biologique mesurée est la température, et le moyen pour régler automatiquement la température est un ventilateur d'extraction (31), placé au dessus du conduit fermé pour la circulation de l'eau, qui est activé par un signal provenant du microprocesseur.

32. Appareil selon la revendication 27,
caractérisé en ce que
la caractéristique physique, chimique ou biologique mesurée est la température, et le moyen pour régler automatiquement la température est une gicleur de gouttes, suspendu au dessus du moyen de culture cellulaire photosynthétique, possédant une soupape qui est activée par un signal provenant du microprocesseur afin de délivrer des gouttes d'eau dans le conduit fermé de circulation d'eau.

33. Appareil selon la revendication 27,
caractérisé en ce que
la caractéristique physique, chimique ou biologique mesurée est la température, et le moyen pour régler automatiquement la température est d'introduire de l'eau froide, d'une température inférieure à celle contenue dans le moyen de culture cellulaire photosynthétique, délivrée dans le conduit fermé de circulation d'eau par l'intermédiaire d'une soupape activée par un signal provenant du microprocesseur.

34. Appareil selon la revendication 27,
caractérisé en ce que
la caractéristique physique, chimique ou biologique mesurée est le rendement des cellules photosynthétiques, et le moyen pour régler automatiquement le rendement s'effectue par la modification du pH, du CO₂, de la température, du contenu en nutriments et de l'introduction d'eau froide dans l'eau circulante.

35. Appareil selon la revendication 15, comprenant de plus un moyen pour purifier et recycler l'eau de culture isolée et séparée des cellules photosynthétiques vers la source d'eau.
